# FASCICULE DE BREVET EUROPEEN

(11) **EP 1 029 059 B1**
(45) Date de publication et mention de la délivrance du brevet: **28.05.2008**
(21) Numéro de dépôt: 98954530.6
(22) Date de dépôt: 06.11.1998
(51) Int. Cl.: C12N 15/53, C12N 15/62, C12N 15/82, C12N 5/10, C12N 9/02, A01H 5/00, A01H 5/10

(54) **HYDROXY-PHENYL PYRUVATE DIOXYGENASE MUTEE, SEQUENCE D'ADN ET OBTENTION DE PLANTES CONTENANT UN TEL GENE, TOLERANTES AUX HERBICIDES**
MUTIERTE HYDROXYPHENYLPYRUVATDIOXYGENASE, DNA SEQUENZ UND HERBIZIDTOLERANTE PFLANZEN, DIE EIN SOLCHES GEN ENTHALTEN
MUTATED HYDROXY-PHENYL PYRUVATE DIOXYGENASE, DNA SEQUENCE AND METHOD FOR OBTAINING HERBICIDE-TOLERANT PLANTS CONTAINING SUCH GENE

(30) Priorité: 07.11.1997 FR 9714264
(43) Date de publication de la demande: 23.08.2000
(73) Titulaire: Bayer CropScience SA, 69009 Lyon (FR)
(72) Inventeur: BOUDEC, Philippe, F-69009 Lyon (FR); BOURDON, Hélène, F-69130 Ecully (FR); DUMAS, Florence, F-69250 Fleurieu sur Saône (FR); RODGERS, Matthew, Ongar, Essex CM5 0HW (GB); SAILLAND, Alain, F-69009 Lyon (FR)
(74) Mandataire: Monconduit, Hervé
(86) Numéro de dépôt international: PCT/FR1998/002374
(87) Numéro de publication internationale: WO 1999/024585

(56) Documents cités:
- WO-A-96/38567
- WO-A-97/27285
- LEE M. ET AL.: "The C-terminal of rat 4-hydroxyphenylpyruvate dioxygenase is indispensable for enzyme activity" FEBS LETTERS, vol. 393, no. 2,3, 16 septembre 1996, pages 269-272, XP002070559
- GARCIA I. ET AL.: "Subcellular localization and purification of a p-hydroxyphenylpyruvate dioxygenase from cultured carrot cells and characterization of the corresponding cDNA" BIOCHEMICAL JOURNAL, (1997 AUG 1) 325 ( PT 3) 761-9. JOURNAL CODE: 9YO. ISSN: 0264-6021., XP002070560
- PADGETTE S R ET AL: "SITE-DIRECTED MUTAGENESIS OF A CONSERVED REGION OF THE 5-ENOLPYRUVYLSHIKIMATE-3-PHOSPHATE SYNTHASE ACTIVE SITE" JOURNAL OF BIOLOGICAL CHEMISTRY, vol. 266, no. 33, 25 novembre 1991, pages 22364-22369, XP002023053

### LISTE DE SEQUENCES

(1) INFORMATIONS GENERALES:
   (i) DEPOSANT:
      (A) NOM: RHONE-POULENC AGROCHIMIE
      (B) RUE: 14-20 Rue Pierre BAIZET
      (C) VILLE: LYON
      (E) PAYS: France
      (F) CODE POSTAL: 69009
   (ii) TITRE DE L'INVENTION: Hydroxy-phényl pyruvate dioxygénase mutée, séquence d'ADN et obtention de plantes contenant un tel gène, tolérantes aux herbicides
   (iii) NOMBRE DE SEQUENCES: 21
   (iv) FORME DECHIFFRABLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 1:
      GAAGTTGCCC TCGCCCCACC CATCGTCGCC CTT 33
(2) INFORMATIONS POUR LA SEQ ID NO: 2:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 2:
      GAAGTTGCCC TCGCCRAKCC CATCGTCGCC CTT 33
(2) INFORMATIONS POUR LA SEQ ID NO: 3:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 3:
      CTTGAAGTTG CCCTCCCAAA ACCCATCGTC GCC 33
(2) INFORMATIONS POUR LA SEQ ID NO: 4:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 4:
      CTTGAAGTTG CCCTCGTCAA ACCCATCGTC GCC 33
(2) INFORMATIONS POUR LA SEQ ID NO: 5:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 5:
      CTTGAAGTTG CCCTCGCTAA ACCCATCGTC GCC 33
(2) INFORMATIONS POUR LA SEQ ID NO: 6:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 6:
      CTTGAAGTTG CCCTCRAKAA ACCCATCGTC GCC 33
(2) INFORMATIONS POUR LA SEQ ID NO: 7:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 7:
      CAGCGCCTTG AAGTTGTCCT CGCCAAACCC ATC 33
(2) INFORMATIONS POUR LA SEQ ID NO: 8:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 8:
      CAGCGCCTTG AAGTTYTCCT CGCCAAACCC ATC 33
(2) INFORMATIONS POUR LA SEQ ID NO: 9:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 9:
      CAGCGCCTTG AAGTTCCACT CGCCAAACCC ATC 33
(2) INFORMATIONS POUR LA SEQ ID NO: 10:
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 10:
      CAGCGCCTTG AAGTTDACTC GCCAAACCCA TC 32
(2) INFORMATIONS POUR LA SEQ ID NO: 11:
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 11:
      CGCTTGAAGT TNNNCTCNNN AAACCCATCG TC 32
(2) INFORMATIONS POUR LA SEQ ID NO: 12:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 12:
      GAACAGCGCC TTGAARAKGC CCTCGCCAAA CCC 33
(2) INFORMATIONS POUR LA SEQ ID NO: 13:
   (i) CARACTERISTIQUES DE LA SEQUENCE:
      (A) LONGUEUR: 33 paires de bases
      (B) TYPE: nucléotide
      (C) NOMBRE DE BRINS: simple
         (D) CONFIGURATION: linéaire
   (ii) TYPE DE MOLECULE: ADN
   (xi) DESCRIPTION DE LA SEQUENCE: SEQ ID NO: 13:
      GCCTTCCACG GAAGATTCGT CCAGCAGGAT ACC 33
(2) INFORMATIONS POUR LA SEQ ID NO: 14:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 14:
      TCGAGAGAGA GGTGACCGAG AGA 23
(2) INFORMATIONS POUR LA SEQ ID NO: 15:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 15:
      TCGATCTCTC GGTCACCTCT CTC 23
(2) INFORMATIONS POUR LA SEQ ID NO: 16:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 16:
      ATTATGGAAT TCGACTATCT T 21
(2) INFORMATIONS POUR LA SEQ ID NO: 17:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 17:
      CAGTATTCAT AATGTTAATT ATG 23
(2) INFORMATIONS POUR LA SEQ ID NO: 18:
   (i) CARACTERISTIQUES DE LA SEQUENCE:
      (A) LONGUEUR: 34 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 18:
      CGGGCAAAAG GATTTARCCA AGGAAACTTT CAAG 34
(2) INFORMATIONS POUR LA SEQ ID NO: 19:
   (i) CARACTERISTIQUES DE LA SEQUENCE:
      (A) LONGUEUR: 34 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 19:
      CGGGCAAAAG GATTTSCNCA AGGAAACTTT CAAG 34
(2) INFORMATIONS POUR LA SEQ ID NO: 20:
   (i) CARACTERISTIQUES DE LA SEQUENCE:
      (A) LONGUEUR: 40 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 20:
      rf2J1Nrf2JNtJNNN GGATCCGGTG CATGGAATTC GACTATCTTC 40
(2) INFORMATIONS POUR LA SEQ ID NO: 21:
   (i) CARACTERISTIQUES DE LA SEQUENCE:
      (A) LONGUEUR: 39 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 21:
      NNNNNNNNNN GGTCACCAGT ATTCATAATG TTAATTATG 39

## Description

La présente invention concerne une séquence d'acide nucléique codant pour une hydroxy-phényl pyruvate dioxygénase (HPPD) mutée, un gène chimère contenant cette séquence comme séquence codante et son utilisation pour l'obtention de plantes résistantes à certains herbicides.

Les hydroxy-phényl pyruvate dioxygénases sont des enzymes qui catalysent la réaction de transformation du para-hydroxy-phényl-pyruvate (HPP) en homogentisate. Cette réaction a lieu en présence de fer (Fe²⁺) en présence d'oxygène (Crouch N.P. & al., Tetrahedron, 53, 20, 6993-7010, 1997). On peut émettre l'hypothèse que les HPPD contiennent un site actif apte à catalyser cette réaction dans lequel viennent se lier le fer, le substrat et la moléculed'oxygène, un tel site actif n'ayant jamais été décrit à ce jour.

On connaît par ailleurs certaines molécules inhibitrices de cette enzyme, qui viennent se fixer à l'enzyme de manière compétitive pour inhiber la transformation de l'HPP en homogentisate. Certaines de ces molécules ont trouvé un emploi comme herbicides, dans la mesure où l'inhibition de la réaction dans les plantes conduit à un blanchiment des feuilles des plantes traitées, et à la mort des dites plantes (Pallett K. E. et al. 1997 Pestic. Sci. 50 83-84). De tels herbicides ayant pour cible l'HPPD décrits dans l'état de la technique sont notamment les isoxazoles (EP 418 175, EP 470 856, EP 487 352, EI- 527 036, EP 560 482, EP 682 659, US 5 424 276) en particulier l'isoxaflutole, herbicide sélectif du maïs, les dicétonitriles (EP 496 630, EP 496 631), en particulier la 2-cyano-3-cyclopropyl-1-(2-SO₂ CH₃-4-CF₃ phényl) propane-1,3-dione et la 2-cyano-3-cyclopropyl-1-(2-SO₂ CH₃-4-2,3 Cl₂ phényl) propane-1, 3-dione, les tricétones (EP 625 505, EP 625 508, US 5,506,195), en particulier la sulcotrione ou encore les pyrazolinates.

Pour rendre les plantes tolérantes aux herbicides, on dispose de trois stratégies principales, (1) la détoxification de l'herbicide par une enzyme venant transformer l'herbicide, ou son métabolite actif, en produits de dégradation non toxique, comme par exemple les enzymes de tolérance au bromoxynil ou au basta (EP 242 236, EP 337 899) ; (2) la mutation de l'enzyme cible en une enzyme fonctionnelle moins sensible à l'herbicide, ou son métabolite actif, comme par exemple les enzymes de tolérance au glyphosate (EP 293 356, Padgette S. R. & al., J. Biol. Chem., 266, 33, 1991) ; ou (3) la surexpression de l'enzyme sensible, de manière à produire dans la plante des quantités suffisantes d'enzyme cible au regard des constantes cinétiques de cette enzyme vis à vis de l'herbicide de manière à avoir suffisamment d'enzyme fonctionnelle, malgré la présence de son inhibiteur.

C'est cette troisième stratégie qui a été décrite pour obtenir avec succès des plantes tolérantes aux inhibiteurs d'HPPD (WO 96/38567), étant entendu que pour la première fois une stratégie de simple surexpression de l'enzyme cible sensible (non mutée) était employée avec succès pour conférer aux plantes une tolérance à un niveau agronomique à un herbicide.

Malgré le succès obtenu avec cette stratégie de simple surexpression de l'enzyme cible, il reste nécessaire d'améliorer le système de tolérance aux inhibiteurs d'HPPD, pour obtenir une tolérance quelques soient les conditions de culture des plantes tolérantes ou les doses commerciales d'application des herbicides dans les champs.

La présente invention concerne donc en premier lieu une HPPD mutée, qui tout en étant fonctionnelle, c'est à dire conservant ses propriétés de catalyse de la transformation de l'HPP en homogentisate, est moins sensible aux inhibiteurs d'HPPD que l'HPPD native, avant mutation.

Au regard du caractère compétitif de l'inhibition, on émet l'hypothèse que les inhibiteurs d'HPPD viennent se lier à l'enzyme dans le site actif de cette dernière, ou à proximité de celui-ci, de manière à bloquer l'accès de l'HPP à ce site actif et empêcher sa transformation en présence de fer etd'oxygène. En effectuant une mutation qui limite l'accès de l'inhibiteur au site actif de l'enzyme tout en préservant l'accès de l'HPP à ce dernier, on peut obtenir des enzymes mutées fonctionnelles, moins sensibles aux inhibiteurs d'HPPD.

On a maintenant constaté qu'en effectuant une mutation de l'enzyme dans sa partie C-terminale, il était possible d'obtenir des HPPD fonctionnelles moins sensibles aux inhibiteurs d'HPPD, de manière que leur expression dans les plantes permette une tolérance améliorée aux inhibiteurs d'HPPD.

La présente invention concerne donc une nouvelle HPPD mutée fonctionnelle, moins sensible aux inhibiteurs d'HPPD, comprenant au moins une mutation dans sa partie C-terminale.

Par « mutation » en entend selon l'invention la substitution d'un acide aminé de la séquence primaire par un autre acide aminé. On emploiera ci-après l'expression « acide aminé muté » pour désigner l'acide aminé remplacé par un autre, désignant le site de la mutation sur la séquence primaire de la protéine.

Plusieurs HPPD et leur séquence primaire ont été décrites dans l'état de la technique, notamment les HPPD de bactéries comme *Pseudomonas* (Rüetschi & al., Eur. J. Biochem., 205, 459-466, 1992, WO 96/38567), de plantes comme d*'Arabidopsis* (WO 96/38567, Genebank AF047834) ou de carotte (WO 96/38567, Genebank 87257), de *Coccicoides* (Genebank COITRP), ou de mammifères comme la souris ou le cochon.

L'alignement de ces séquences connues, par les moyens usuels de la technique, comme par exemple la méthode décrite par Thompson, J.D. & al. (CLUSTAL W: improving the sensitivity of progressive multiple séquence alignment through séquence weighting, positions-specific gap penalties and weight matrix choice. Nucleic Acids Research, 22:4673-4680, 1994), et l'accès à ces programmes informatiques d'alignement de séquences accessibles par exemple via internet, l'homme du métier peut définir les homologies de séquence par rapport à une séquence de référence, et retrouver les acides aminés clés, ou encore définir des régions communes, notamment permettant de définir une région C-terminale et une région N-terminale à partir de cette séquence de référence.

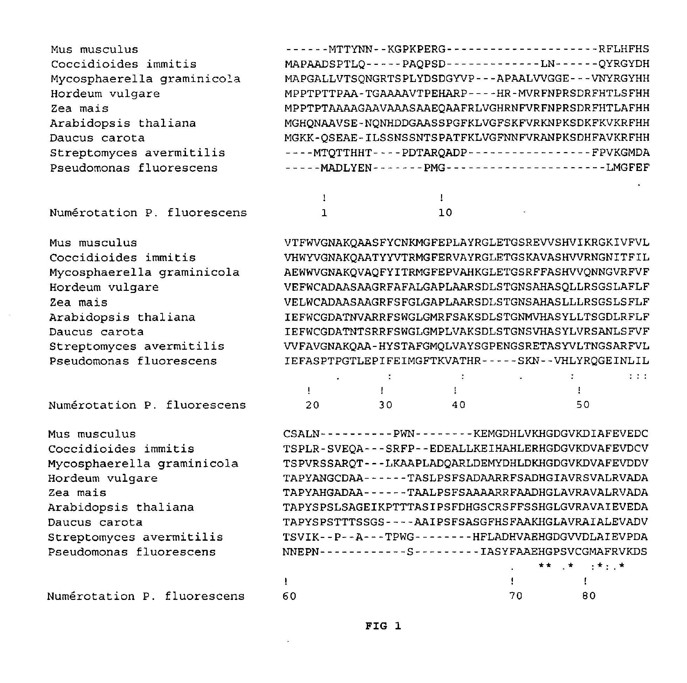

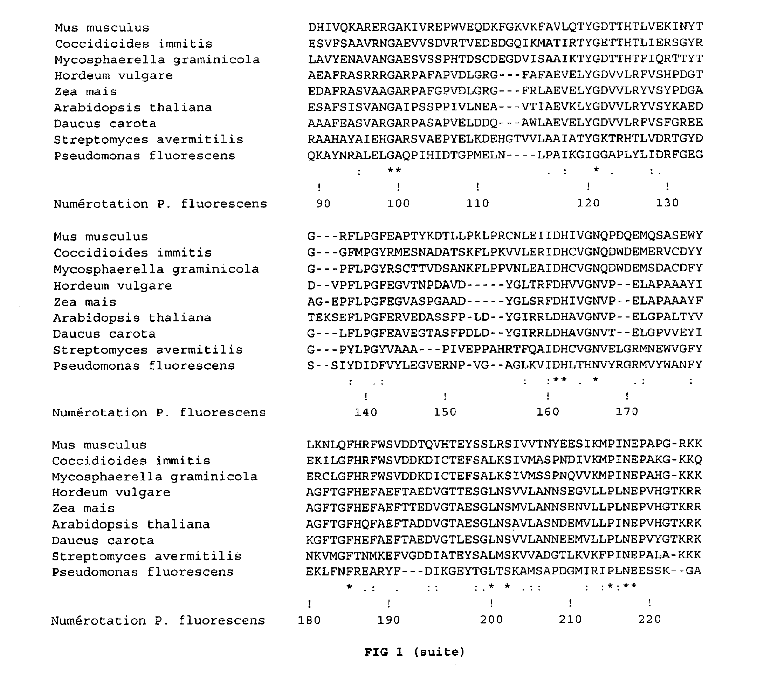

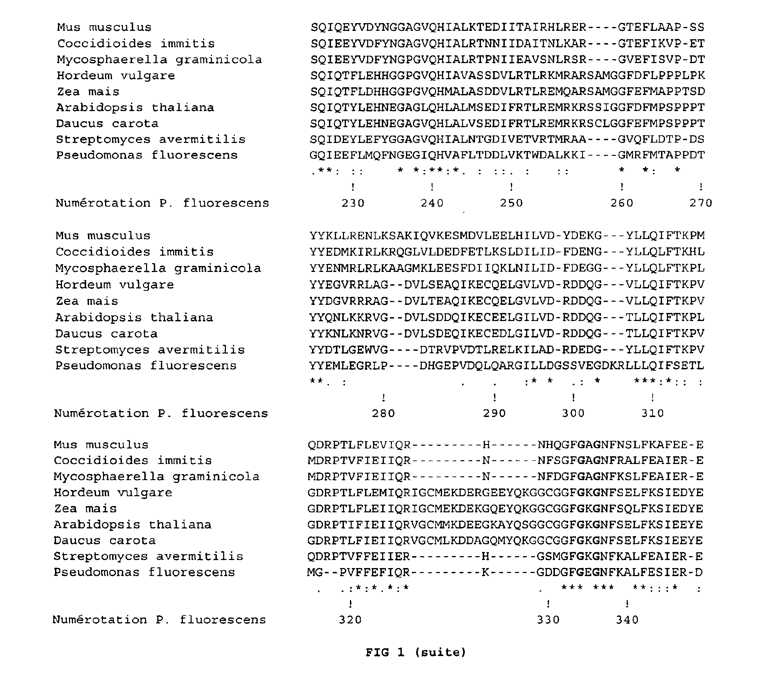

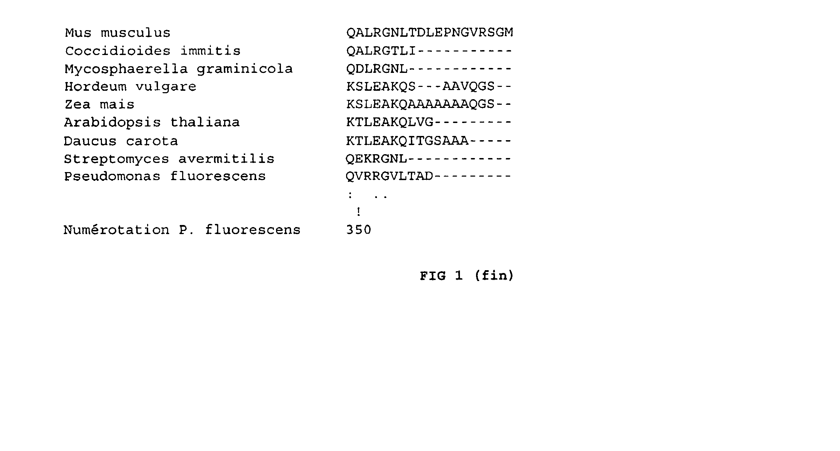

Pour la présente invention, la séquence de référence est la séquence de *Pseudomonas,* toutes les définitions et indications de positions d'acides aminés particuliers étant faites par rapport à la séquence primaire d'HPPD de *Pseudomonas.* La figure 1 en annexe représente un alignement de plusieurs séquences d'HPPD décrites dans l'état de la technique, alignées par rapport à la séquence d'HPPD de *Pseudomonas* comme référence, comprenant les séquence d'HPPD de *Streptomyces avermitilis* (Genebank SAV11864), de *Daucus carota* (Genebank DCU 87257), *d'Arabidopsis thaliana* (Genebank AF047834), de *Zea mais,* de *Hordeum vulgare* (Genebank HVAJ693), de *Mycosphaerella graminicola* (Genebank AF038152), de *Coccicoides immitis* (Genebank COITRP) et de *Mus musculus* (Genebank MU54HD). La numérotation des acides aminés de la séquence de *Pseudomonas* est donnée sur cette figure, de même que les acides aminés communs à ces séquences, désignés par une astérisque. Sur la base d'un tel alignement il est aisé d'identifier à partir de la définition de l'acide aminé de *Pseudomonas* par sa position et sa nature, la position de l'acide aminé correspondant dans une autre séquence d'HPPD (l'alignement de séquences de différentes origines, plantes, mammifères, bactéries, montrant que cette méthode d'alignement bien connue de l'homme du métier peut être généralisée à toute autre séquence). Un alignement de différentes séquences d'HPPD est également décrit dans la demande de brevet WO 97/49816.

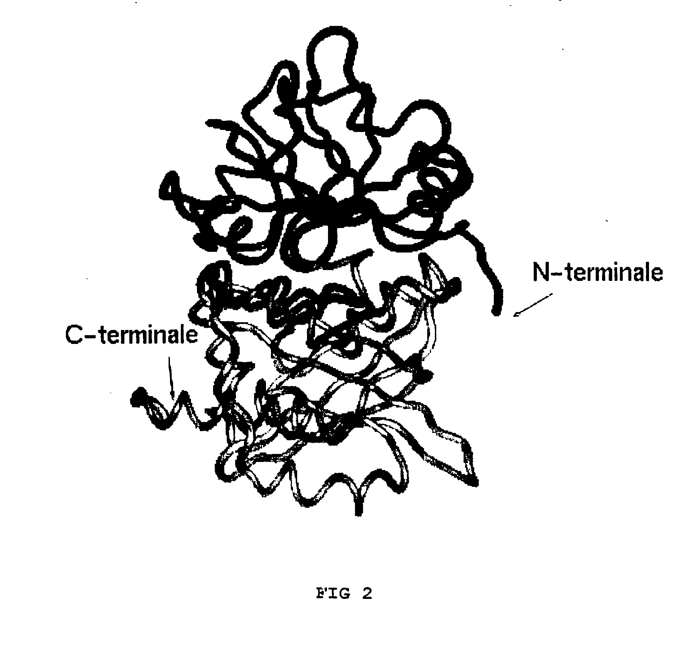

La partie C-terminale des HPPD, siège du site actif de l'enzyme, se distingue par un peptide de liaison de sa partie N-terminale, assurant la stabilité de l'enzyme et son oligomérisation (l'HPPD de *Pseudomonas* est un tétramère, celles de plantes sont des dimères) comme le montre la représentation schématique de la structure ternaire du monomère de l'HPPD de *Pseudomonas* représentée en figure 2. Cette structure a été obtenue par les méthodes usuelles d'étude de la diffraction aux rayons X de cristaux. Le peptide de liaison va permettre de définir l'extrémité N-terminale de la partie C-terminale de l'enzyme, ledit peptide se situant entre les acides aminés 145 et 157 pour *Pseudomonas* (c.f. figure 1).

La partie C-terminale peut donc être définie comme constituée par la séquence définie d'une part par le peptide de liaison, et d'autre part par l'extrémité C-terminale de l'enzyme, la mutation effectuée dans la partie C-terminale d'HPPD étant donc effectuée dans la zone ainsi définie. Sur l'alignement de séquences représenté sur la figure 1 en annexe, on remarque pour toutes les séquences deux acides aminés en position 161 et 162 pour la séquence de *Pseudomonas,* (D = Asp161 et H = His162). Par référence à l'HPPD de *Pseudomonas,* on peut donc définir que le peptide de liaison représentant l'extrémité N-terminale de la partie C-terminale de l'HPPD se situe entre environ 5 et 15 acides aminés en amont de l'acide aminé Asp161.

Selon un mode préférentiel de réalisation de l'invention, la mutation est effectuée sur des acides aminés, qui sont remplacés par des acides aminés d'encombrement stérique supérieur ou encore un des acides aminés ionisés ou ionisables. De préférence, la mutation est effectuée sur des acides aminés de faible encombrement stérique. Par acide aminé de faible encombrement stérique, on entend de préférence selon l'invention la glycine ou les acides aminés d'encombrement stérique faible comme l'alanine, la cystéine, la sérine....

Tout acide aminé d'encombrement stérique supérieur à celui de l'acide aminé remplacé peut être employé pour la mutation selon l'invention. De manière préférentielle, on remplacera les acides aminés du site de mutation par les acides aminés suivants : leucine, isoleucine ou tryptophane.

Par acide aminé ionisé ou ionisable, on entend selon l'invention tout acide aminé présentant outre les groupes entrant dans la liaison peptidique, un groupe amino, acide carboxylique (COOH), ou encore ammonium ou -COO-. Il s'agit de préférence des acides aminés suivants : glutamine et acide glutamique ou asparagine ou acide aspartique.

Selon un mode préférentiel de réalisation de l'invention, la mutation sera effectuée sur un acide aminé de la partie C-terminale commun à plusieurs séquences d'HPPD, ces derniers pouvant être identifiés par la méthode d'alignement de séquence.

Selon un mode particulier de réalisation de l'invention, l'HPPD mutée comprend dans sa partie C-terminale, la séquence peptidique suivante :
- Gly - Phe - Xaa - Yaa - Xab - Asn - Phe - Yab - Yac - Leu - Phe -
dans laquelle Xaa et Xab représentent indépendamment l'un de l'autre la glycine (Gly) ou un acide aminé d'encombrement supérieur à la glycine, étant entendu que si l'un de Xaa ou Xab représente Gly, alors l'autre est différent de Gly,
Yaa représente un acide aminé quelconque, de préférence Ala, Lys ou Glu*,
Yab représente un acide aminé quelconque, de préférence Lys, Ser, Arg ou Asn*, et
Yac représente un acide aminé quelconque, de préférence Ala, Ser, Glu ou Gln*.

De manière avantageuse, l'un au moins de Xaa ou Xab représente Leu, Glu, Trp ou Ile.

Par référence à la séquence d'HPPD de *Pseudomonas,* les acides aminés mutés sont choisis parmi les acides aminés suivants : Pro215, Gly298, Gly332, Phe333 Gly334 Gly336 et Asn337, plus préférentiellement les acides aminés Pro215 et Gly336.

Parmi les exemples de mutations préférées, on citera les mutations suivantes Pro215Leu, Gly336Glu, Gly336Trp ou Gly336Ile.

Il est entendu que les mutations décrites ci-dessus peuvent être combinées deux à deux, comme par exemple une double mutation des acides aminés Gly334 et Gly336. La présente invention concerne également une séquence d'acide nucléique codant pour une HPPD mutée décrite ci-dessus. Selon la présente invention, on entend par « séquence d'acide nucléique » une séquence nucléotidique pouvant être de type ADN ou ARN, de préférence de type ADN, notamment double brin, qu'elle soit d'origine naturelle ou synthétique, notamment une séquence d'ADN pour laquelle les codons codant pour l'HPPD mutée selon l'invention auront été optimisés en fonction de l'organisme hôte dans lequel elle sera exprimée, ces méthodes d'optimisations étant bien connues de l'homme du métier.

La séquence codant pour une HPPD d'origine non muté, peut. être d'origine quelconque. En particulier elle peut être d'origine bactérienne. Comme exemples intéresants on peut citer des bactéries du type *Pseudomonas sp,* par exemple *Pseudomonas fluorescens* ou encore des cyanobactéries du type *Synechocystis.* La séquence peut être aussi d'origine végétale, notamment issu de plantes dicotyledones telles que tabac, *Arabidopsis,* d'ombellifères telles que *Daucus carotta* ou encore monocotyledones telles que le *Zea maïs* ou le blé ou bien encore l'orge. Les séquences codantes et le moyen de les isoler et cloner sont décrits dans les références citées auparavant, dont le contenu est incorporé ici par référence.

La mutation pourra être effectuée dans la séquence d'acide nucléique codant pour l'HPPD d'origine non mutée par tout moyen approprié pour remplacer dans ladite séquence le codon codant pour l'acide aminé muté par le codon correspondant à l'acide aminé venant le remplacer, lesdits codons étant largement décrits dans la littérature et bien connus de l'homme du métier.

Plusieurs procédés de la biologie moléculaire permettent de réaliser cette mutation.

Un premier procédé consiste à soumettre des cultures de cellules à une pression de sélection longue avec un inhibiteur de l'HPPD, en présence ou non d'un agent mutagène, le gène de l'HPPD mutant alors spontanément sous l'effet de cette pression de sélection, et éventuellement de l'agent mutagène, ledit gène ayant évolué de telle sorte qu'il code pour une enzyme mutée permettant l'expression de l'activité HPPD dans des conditions où l'enzyme non modifiée est inhibée en partie ou en totalité. Les cellules peuvent être des cellules végétales ou des bactéries, et dans ce deuxième cas, elles peuvent exprimer une HPPD native (d'origine bactérienne) ou une HPPD d'une autre origine (bactérienne, champignons, algues, plantes), introduite dans la bactérie employée pour la mutagénèse sous une forme appropriée permettant l'expression de cette HPPD, le gène codant pour l'HPPD native de ladite bactérie ayant été de préférence supprimé s'il existe. De tels moyens de transformation des bactéries sont bien connus de l'homme du métier, abondamment décrits dans la littérature, de même que les moyens de mutation (notamment : Sambrook & al., Molecular Cloning : A Laboratory Manual, Second Edition, Cold Spring Harbor Laboratory Press, Cold Spring Harbor, New York).

Dans le cas où la cellule est une cellule végétale exprimant une HPPD native, l'HPPD mutée peut être isolée et clonée, ou encore des plantes peuvent être régénérées à partir des cultures cellulaires selon les méthodes usuelles. Les plantes ainsi obtenues expriment alors une HPPD mutée fonctionnelle moins sensible aux inhibiteurs d'HPPD que l'HPPD native. Les méthodes de régénérations sont abondamment décrites dans la littérature (y compris dans les références citées précédemment) et bien connues de l'homme du métier.

La sélection des cellules présentant une HPPD mutée moins sensible aux inhibiteurs d'HPPD est effectuée au moyen d'un outil de criblage approprié. Au regard de l'objet de la présente invention et de la solution recherché, une HPPD moins sensibles aux inhibiteurs d'HPPD, un outil de criblage simple à mettre en oeuvre consiste à déterminer les doses d'inhibiteur d'HPPD qui inhibent à 100 % les HPPD d'origine non mutées, létale pour les cellules exprimant cette HPPD non mutée, de soumettre les cellules après mutation à cette dose déterminée, d'isoler les cellules mutées ayant résistées à cette dose létale, puis d'isoler et de cloner le gène codant pour l'HPPD mutée.

Cette opération de mutagénèse par culture cellulaire a été effectuée sur un nombre très important de cellules de *Pseudomonas* exprimant leur HPPD native (voir notamment les exemples particuliers décrits plus loin). Dans tous les cas, les mutants isolés par la méthode de sélection définie ci-dessus ont été des mutants présentant une mutation dans la partie C-terminale de l'HPPD.

Un autre procédé de préparation d'une séquence d'acide nucléique mutée selon l'invention, et de la protéine correspondante consiste à effectuer une mutagénèse dirigée sur un ou plusieurs acides aminés sélectionnés à l'avance, par exemple en identifiant les acides aminés communs à plusieurs séquences dans la partie C-terminale, ou encore en cherchant à reproduire dans une HPPD d'une origine déterminée, une mutation obtenue par mutagénèse au hasard (culture de cellule) dans une HPPD d'une autre origine. Les moyens d'obtenir ces mutations dirigées sont bien connus de l'homme du métier et largement décrits dans la littérature (notamment : Directed Mutagenesis : A Practical Approach, 1991, Edited by M.J. McPHERSON, IRL PRESS), ou pour lesquels on peut employer des kits commerciaux (par exemple l'U.S.E. Mutagenesis Kit de la société PHARMACIA). Dans tous les cas, il est utile d'employer après cette mutagénèse dirigée la même méthode de sélection des HPPD mutées moins sensibles que l'HPPD non mutée correspondante employée pour la mutagénèse au hasard décrite ci-dessus.

La présente invention concerne donc également un procédé de préparation d'une séquence d'acide nucléique codant pour une HPPD mutée selon l'invention; ledit procédé étant défini ci-dessus.

L'invention a encore pour objet l'utilisation d'une séquence d'acide nucléique codant pour une HPPD mutée selon l'invention dans un procédé pour la transformation des plantes, comme gène marqueur ou comme séquence codante permettant de conférer à la plante une tolérance aux herbicides inhibiteurs d'HPPD. Cette séquence peut bien entendu également être utilisée en association avec d'autre(s) gène(s) marqueur(s) et/ou séquence(s) codante(s) pour une ou plusieurs propriétés agronomiques.

La présente invention concerne également un gène chimère (ou cassette d'expression) comprenant une séquence codante ainsi que des éléments de régulation en position 5' et 3' hétérologues pouvant fonctionner dans un organisme hôte, en particulier les cellules végétales ou les plantes, la séquence codante comprenant au moins une séquence d'acide nucléique codant pour une HPPD mutée telle que définie précédemment.

Par organisme hôte, on entend tout organisme mono ou pluricellulaire, inférieur ou supérieur, dans lequel le gène chimère selon l'invention peut être introduit, pour la production d'HPPD mutée. Il s'agit en particulier de bactéries, par exemple *E. coli,* de levures, en particulier des genres *Saccharomyces* ou *Kluyveromyces, Pichia,* de champignons, en particulier *Aspergillus,* d'un baculovirus, ou de préférence des cellules végétales et des plantes.

Par "cellule végétale", on entend selon l'invention toute cellule issue d'une plante et pouvant constituer des tissus indifférenciés tels que des cals, des tissus différenciés tels que des embryons, des parties de plantes, des plantes ou des semences.

On entend par "plante" selon l'invention, tout organisme multicellulaire différencié capable de photosynthèse, en particulier monocotylédones ou dicotylédones, plus particulièrement des plantes de culture destinées ou non à l'alimentation animale ou humaine, comme le maïs, le blé, le colza, le soja, le riz, la canne à sucre, la betterave, le tabac, le coton, etc.

Les éléments de régulation nécessaires à l'expression de la séquence d'acide nucléique codant pour une HPPD sont bien connus de l'homme du métier en fonction de l'organisme hôte. Ils comprennent notamment des séquences promotrices, des activateurs de transcription, des séquences terminatrices, y compris des codons start et stop. Les moyens et méthodes pour identifier et sélectionner les éléments de régulation sont bien connus de l'homme du métier et largement décrits dans la littérature.

L'invention concerne plus particulièrement la transformation des plantes. Comme séquence de régulation promotrice dans les plantes, on peut utiliser toute séquence promotrice d'un gène s'exprimant naturellement dans les plantes en particulier un promoteur s'exprimant notamment dans les feuilles des plantes, comme par exemple des promoteurs dits constitutifs d'origine bactérienne, virale ou végétale tel que, par exemple un promoteur d'histone tel que décrit dans la demande EP 0 507 698, ou un promoteur d'actine de riz. d'un gène de virus de plante tel que, par exemple, celui de la mosaïque du choux fleur (CAMV 19S ou 35S), ou encore des promoteurs dits lumière dépendants comme celui d'un gène de la petite sous-unité de ribulose-biscarboxylase/oxygénase (RuBisCO) de plante, ou tout promoteur convenable connu pouvant être utilisé.

Selon l'invention, on peut également utiliser, en association avec la séquence de régulation promotrice, d'autres séquences de régulation, qui sont situées entre le promoteur et la séquence codante, telles que des activateurs de transcription ("enhancer"), comme par exemple l'activateur de translation du virus de la mosaïque du tabac (TMV) décrit dans la demande WO 87/07644, ou du virus etch du tabac (TEV) décrit par Carrington & Freed.

Comme séquence de régulation terminatrice ou de polyadénylation, on peut utiliser toute séquence correspondante d'origine bactérienne, comme par exemple le terminateur nos *d'Agrobacterium tumefaciens,* ou encore d'origine végétale, comme par exemple un terminateur d'histone tel que décrit dans la demande EP 0 633 317.

Selon un mode particulier de réalisation de l'invention, on emploie en 5' de la séquence d'acide nucléique codant pour une HPPD mutée, une séquence d'acide nucléique codant pour un peptide de transit, cette séquence étant disposée entre la région promotrice et la séquence codant pour l'HPPD mutée de manière à permettre l'expression d'une protéine de fusion peptide de transit/HPPD mutée, cette dernière étant définie précédemment. Le peptide de transit permet d'adresser l'HPPD mutée dans les plastes, plus particulièrement les chloroplastes, la protéine de fusion étant clivée entre le peptide de transit et l'HPPD mutée au passage de la membrane des plastes. Le peptide de transit peut être simple, comme un peptide de transit d'EPSPS (décrit dans le brevet US 5,188,642) ou un peptide de transit de celui de la petite sous-unité de ribulose-biscarboxylase/oxygénase ( ssu RuBisCO) d'une plante, éventuellement comprenant quelques acides aminés de la partie N-terminale de la ssu RuBisCO mature (EP 189 707) ou encore un peptide de transit multiple comprenant un premier peptide de transit de plante fusionné à une partie de la séquence N-terminale d'une protéine mature à localisation plastidiale, fusionnée à un deuxième peptide de transit de plante tel que décrit dans le brevet EP 508 909, et plus particulièrement le peptide de transit optimisé comprenant un peptide de transit de la ssu RuBisCO de tournesol fusionné à 22 acides aminés de l'extrémité N-terminale de la ssu RBisCO de maïs fusionnée au peptide de transit de la ssu RuBisCO de maïs tel que décrit avec sa séquence codante dans le brevet EP 508 909.

La présente invention concerne également la protéine de fusion peptide de transit/HPPD mutée, les deux éléments de cette protéine de fusion étant définis plus haut.

La présente invention concerne également un vecteur de clonage et/ou d'expression pour la transformation d'un organisme hôte contenant au moins un gène chimère tel que défini ci-dessus. Ce vecteur comprend outre le gène chimère ci-dessus, au moins une origine de réplication. Ce vecteur peut être constitué par un plasmide, un cosmide, un bactériophage ou un virus, transformés par l'introduction du gène chimère selon l'invention. De tels vecteurs de transformation en fonction de l'organisme hôte à transformer sont bien connus de l'homme du métier et largement décrits dans la littérature. Pour la transformation des cellules végétales ou des plantes, il s'agira notamment d'un virus qui peut être employé pour la transformation des plantes développées et contenant en outre ses propres éléments de réplication et d'expression. De manière préférentielle, le vecteur de transformation des cellules végétales ou des plantes selon l'invention est un plasmide.

L'invention a encore pour objet un procédé de transformation des organismes hôtes, en particulier des cellules végétales par intégration d'au moins une séquence d'acide nucléique ou un gène chimère tels que définis ci-dessus, transformation qui peut être obtenue par tout moyen connu approprié, amplement décrit dans la littérature spécialisée et notamment les références citées dans la présente demande, plus particulièrement par le vecteur selon l'invention.

Une série de méthodes consiste à bombarder des cellules, des protoplastes ou des tissus avec des particules auxquelles sont accrochées les séquences d'ADN. Une autre série de méthodes consiste à utiliser comme moyen de transfert dans la plante un gène chimère inséré dans un plasmide Ti *d'Agrobacterium tumefaciens* ou Ri *d'Agrobticterium rhizogenes.* D'autres méthodes peuvent être utilisées telles que la riticro-injection OU l'électroporation, ou encore la précipitation directe au moyen de PEG. L'homme du métier fera le choix de la méthode appropriée en fonction de la nature de l'organisme hôte, en particulier de la cellule végétale ou de la plante.

La présente invention a encore pour objet les organismes hôtes, en particulier cellules végétales ou plantes, transformés et contenant un gène chimère comprenant une séquence codante pour une HPPD mutée définie ci-dessus.

La présente invention a encore pour objet les plantes contenant des cellules transformées, en particulier les plantes régénérées à partir des cellules transformées. La régénération est obtenue par tout procédé approprié qui dépend de la nature de l'espèce, comme par exemple décrit dans les références ci-dessus. Pour les procédés de transformation des cellules: végétales et de régénération des plantes, on citera notamment les brevets et demandes de brevet suivants: US 4,459,355, US 4,536,475, US 5,464,763, US 5,177,010, US 5,187,073, EP 267,159, EP 604 662, EP 672 752, US 4,945,050, US 5,036,006, US 5,100,792, US 5,371,014, US 5,478,744, US 5,179,022, US 5,565,346, US 5,484,956, US 5,508,468, US 5,538,877, US 5,554,798, US 5,489,520, US 5,510,318, US 5,204,253, US 5,445,765, EP 442 174, EP 486 233, EP 486 234, EP 539 563, EP 674 775, WO 91/02071 et WO 95/06128.

La présente invention concerne également les plantes transformées issues de la culture et/ou du croisement des plantes régénérées ci-dessus, ainsi que les graines de plantes transformées contenant des cellules transformées.

Les plantes transformées pouvant être obtenues selon l'invention peuvent être du type monocotylédones telles que par exemple les, céréales, la canne à sucre, le riz et le maïs ou dicotylédones comme par exemple le tabac, la soja, le colza, le coton, la betterave, le trèfle, etc.

L'invention a aussi pour objet un procédé de désherbage sélectif de plantes, notamment de cultures, à l'aide d'un inhibiteur de l'HPPD notamment un herbicide définit auparavant, caractérisé en ce qu'on applique cet herbicide sur des plantes transformées selon l'invention, tant en présemis, en prélevée qu'en postlevée de la culture.

La présente invention concerne également un procédé de contrôle des mauvaises herbes dans une surface d'un champ comprenant des graines ou des plantes transformées avec le gène chimère selon l'invention, lequel procédé consiste à appliquer dans la dite surface du champ une dose toxique pour les dites mauvaises herbes d'un herbicide inhibiteur d'HPPD, sans toutefois affecter de manière substantielle les graines ou plantes transformée avec le dit gène chimère selon l'invention.

La présente invention concerne également un procédé de culture des plantes transformées selon l'invention avec un gène chimère selon l'invention lequel procédé consiste à semer les graines des dites plantes transformées dans une surface d'un champ approprié pour la culture des dites plantes, à appliquer sur la dite surface du dit champ une dose toxique pour les mauvaises herbes d'un herbicide ayant pour cible l'HPPD défini ci-dessus en cas de présence de mauvaises herbes, sans affecter de manière substantielle les dites graines ou les dites plantes transformées, puis à récolter les plantes cultivées lorsqu'elles arrivent à la maturité souhaitée et éventuellement à séparer les graines des plantes récoltées.

Dans les deux procédés ci-dessus, l'application de l'herbicide ayant pour cible l'HPPD peut être faite selon l'invention, tant en présemis, en prélevée qu'en postlevée de la culture.

Par herbicide au sens de la présente invention on entend une matière active herbicide seule ou associée à un additif qui modifie son efficacité comme par exemple un agent augmentant l'activité (synergiste) ou limitant l'activité (en anglais safener). Les herbicides inhibiteurs d'HPPD sont en particulier définis auparavant. Bien entendu, pour leur application pratique, les herbicides ci-dessus sont associée de manière en soi connue aux adjuvants de formulations utilisés habituellement en agrochimie

Lorsque la plante transformée selon l'invention comprend un autre gène de tolérance à un autre herbicide (comme par exemple un gène codant pour une EPSPS mutée ou non conférant à la plante une tolérance au glyphosate), ou lorsque la plante transformée est naturellement insensible à un autre herbicides, le procédé selon l'invention peut comprendre l'application simultanée ou décalée dans le temps d'un inhibiteur d'HPPD en association avec ledit herbicide, par exemple le glyphosate.

L'invention a encore pour objet l'utilisation du gène chimère codant pour une HPPD mutée comme gène marqueur au cours du cycle "transformation-régénération" d'une espèce végétale et sélection sur l'herbicide ci-dessus

Les différents aspects de l'invention seront mieux compris à l'aide des exemples expérimentaux ci-dessous.

Toutes les méthodes ou opérations décrites ci-dessous dans ces exemples sont données à titre d'exemples et correspondent à un choix, effectué parmi les différentes méthodes disponibles pour parvenir au même résultat. Ce choix n'a aucune incidence sur la qualité du résultat et par conséquent, toute méthode adaptée peut être utilisée par l'homme de l'art pour parvenir au même résultat. La plupart des méthodes d'ingénierie des fragments d'ADN sont décrites dans "Current Protocols in Molecular Biology" Volumes 1 et 2, Ausubel F.M. et al , publiés par Greene Publishing Associates et Wiley -Interscience (1989) ou dans Molecular cloning, T.Maniatis, E.F.Fritsch, J.Sambrook,1982.

### Exemple 1 : Test de criblage colorimétrique de mutants présentant une tolérance à la 2-cyano-3-cyclopropyl-1-(2-SO₂CH₃-4-CF₃ phényl) propane-1,3-dione

pRP C : Le vecteur pRP A (décrit dans la demande WO 96/38567) contenant un fragment d'ADN génomique et la région codante du gène de l'HPPD de *Pseudomonas fluorescens* A32 a été digéré par NcoI, purifié puis ligué dans le vecteur d'expression pKK233-2 (Clontech) lui-même digéré par NcoI, site de clonage unique de ce vecteur. L'orientation du gène dans le vecteur pRP C ainsi obtenu, permettant l'expression sous le' contrôle du promoteur *trc* aété vérifiée.

Un milieu de culture de type YT broth à 1% d'agarose (ultra pur Gibco BRL) et à 5mM de L-Tyrosine (Sigma), et contenant l'agent de sélection du vecteur pRP C ci-dessus est dispensé en plaque de 96 puits à raison de 100ul par puits. 10ul d'une culture de E.Coli en phase exponentielle de croissance contenant le vecteur pRP C sontdispensés dans chaque puits. Après 16 heures à 37°C, les puits ne contenant que le milieu de culture ou ceux ensemencés avec une culture d'E.Coli contenant le vecteur PKK233-2 sont translucides, alors que les puits ensemencés avec une culture de E.Coli contenant le vecteur pRP C sont de couleur brune.

Une gamme a été réalisée avec milieu de culture identique contenant des concentrations variables (0mM à 14mM) du 2-cyano-3-cyclopropyl-1-(2-méthylsulfonyl-4-trifluorométhylphényl) propan-1,3-dione (EP 0 496 631) solubilisé dans l'eau et amené à pH7,5. Cette molécule est un dicétonitrile, reconnu comme étant un inhibiteur efficace d'activité HPPD (Pallett, K.E. et al 1997. Pestic. Sci. 50, 83-84). On observe une absence totale de coloration pour la culture bactérienne contenant le vecteur pRP C à 7mM du composé ci-dessus.

Des mutants d'HPPD obtenus par mutagenèse dirigée aussi bien que par mutagenèse au hasard ont été sélectionnés par brunissement du milieu contenant du 2-cyano-3-cyclopropyl-1-(2-méthylsulfonyl-4-trifluorométhylphényl) propan-1,3-dione à la concentration de 7mM comme cela sera montré ci-après.

Des résultats identiques ont été obtenus en substituant, à la 2-cyano-3-cyclopropyl-1-(2-méthyl-4-trifluorométhylphényl)propan-1,3-dione, la 2-cyano-3-cyclopropyl-1-(2-méthylsulfonyl -4-(méthylthio) phényl) propan-1,3-dione, et la 2-(2-chloro-3-ethoxy-4-(éthylsulfonyl) benzoyl)-5-méthyl-1,3-cyclohexadione,(WO 93/03009). utilisées respectivement à 3,5mM et 7mM.

Ces résultats confirment qu'un test basé sur l'activité HPPD quelle que soit l'origine de cette activité, permet d'identifier des activités HPPD présentant une tolérance à des inhibiteurs d'activité HPPD de famille des isoxazoles aussi bien que des tricétones.

### Exemple 2: Mutagenèse au hasard du gène de l'HPPD de Pseudomonas fluorescens A32 à l'aide de l'hydroxylamine

L'ADN plasmidique d'une culture de *E.Coli* contenant le vecteur pRP C décrit ci-dessus a été extrait en utilisant le protocole standard. Cet ADN a été mis en présence d'hydroxylamine, agent chimique mutagène provoquant la substitution de la Cytosine en Thymidine, pendant une heure à 80°C, en utilisant un protocole standard. La souche DH10B de *E.Coli.* K12 a été transformée avec l'ADN plasmidique potentiellement muté ainsi obtenu. L'utilisation du test de criblage colorimétrique décrit dans l'exemple 1 a permis d'identifier après criblage de plusieurs milliers de clones potentiellement mutés plusieurs colonies capables de brunir le milieu, c'est à dire de transformer l'HPP en homogentisate même en présence de 7mM de 2-cyano-3-cyclopropyl-1-(2-méthylsulfonyl-4-trifluorométhylphényl) propan-1,3-dione .

Après séquençage de ces divers mutants il s'est révélé que l'utilisation de cet outil de mutagenèse/criblage a permis d'isoler 4 mutants distincts correspondants à trois sites de mutations. distincts:
- Premier site: la proline 215 remplacée par une leucine, mutant appelé PfL215
- Deuxième site:
   - la glycine 334 remplacée par une sérine, mutant appelé PfS334
   - la glycine 334 remplacée par un acide aspartique, mutant appelé PfD334
- Troisième site: la glycine 336 remplacée par un acide sérine, mutant appelé PfS336

Ces trois sites mutés permettant d'obtenir une tolérance améliorée par rapport à l'HPPD non mutée sont situés dans la partie C-terminale de la protéine.

Les clones dérivés du clone pRP C contenant la région codante du gène de l'HPPD de Pseudomonas fluorescens A32 contenant une ou plusieurs mutations sont nommés pRP C suivit du nom de la ou des mutations effectuées, comme par exemple pRP C PfL215 ou pRPC PIS336 (ou encore simplement par la seule désignation de l'acide aminé muté, comme par exemple PfL215 ou PfS336, sauf indication contraire quant à l'origine de l'HPPD mutée).

La ou les modifications par mutagenèse au hasard peuvent être effectuées sur toute protéine ayant une activité de type HPPD c'est à dire transformant le 4-hydroxy phénylpyruvate en homogentisate, dont la région codante est ou pourrait être clonée. Les HPPD décrites dans ce texte sont entre autres celle de *P. fluorescens, Arabidopsis thaliana, Daucus carota, Zea mais et Synechocystis,* mais bien sûr il est évident pour l'homme de métier que toutes ces modifications sont applicables aux autres HPPD.

### Exemple 3: Mutagenèse dirigée du gène de l'HPPD de Pseudomonas fluorescens A32 par analogie de séquence.

Par alignement des séquences protéiques des HPPD de *Pseudomonas _{f}luorescens* A32, *Arabidopsis thaliana,* de souris de cochon et de *Coccicoides...,* il est possible de choisir parmi les acides aminés que l'on retrouve conservés dans différentes séquences un certain nombre d'entre eux pour les mutagénéiser et obtenir des HPPD tolérantes; on aurait pu ajouter à l'alignement , présenté en figure 1, des séquences d'autres HPPD décrits dans la littérature.

L'alignement de ces différentes séquences met nettement en évidence qu'une des régions les mieux conservées est située entre la glycine en position 332 et la phénylalanine 342. Non seulement cette région est très conservée mais en plus elle englobe les deux glycines en position 334 et 336 identifiées par mutagenèse au hasard (en gras sur l'alignement de séquences et marqués d'une étoile). On a réalisé la mutagenèse sur le vecteur pRP C en utilisant l'U.S.E. Mutagenesis Kit, Pharmacia. Les oligonucléotide Mont été utilisés pour les mutagenèses de la phénylalanine 333, de la glycine 334, de la glycine 336 et de l'asparagine 337, mais aussi pour celles des doubles mutants des glycines 334 et 336, comme indiqué dans les schémas ci-après (identificateurs de séquences en annexe, SEQ ID NO 1 à 12) :
Mutagenèse de PHE333, remplacée uniquement par Trp
Oligo 1 : GAAGTTGCCC TCGCCCCACC CATCGTCGCC CTT
Mutagenèse de PHE333, remplacée uniquement par LEU & ILE
Oligo 2 : GAAGTTGCCC TCGCCRA**K**CC CATCGTCGCC CTT
Mutagenèse de GLY334, remplacée uniquement par TRP
Oligo 3 : CTTGAAGTTG CCCTCCCAAA ACCCATCGTC GCC
Mutagenèse de GLY334, remplacée uniquement par ASP
Oligo 4 : CTTGAAGTTG CCCTCGTCAA ACCCATCGTC GCC
Mutagenèse de GLY334, remplacée uniquement par SER
Oligo 5 : CTTGAAGTTG CCCTCGCTAA ACCCATCGTC GCC
Mutagenèse de GLY334, remplacée uniquement par LEU & ILE
Oligo 6 : CTTGAAGTTG CCCTC**R**A**K**AA ACCCATCGTC GCC
Mutagenèse de GLY336, remplacée uniquement par ASP
Oligo 7 : CAGCGCCTTG AAGTTGTCCT CGCCAAACCC ATC
Mutagenèse de GLY336, remplacée uniquement par GLU
Oligo 8 : CAGCGCCTTG AAGTT**Y**TCCT CGCCAAACCC ATC
Mutagenèse de GLY336, remplacée uniquement par TRP
Oligo 9 : CAGCGCCTTG AAGTTCCACT CGCCAAACCC ATC
Mutagenèse de GLY336, remplacée uniquement par Ile
Oligo 10 : CAGCGCCTTG AAGTT**D**ACTC GCCAAACCCA TC -
Mutagenèse de GLY334 & GLY336, remplacées par tous les autres acides aminés, donc avec possibilité d'obtenir un double mutant
Oligo 11 : CGCTTGAAGT T**NNN**CTC**NNN** AAACCCATCG TC
Mutagenèse de ASN337, remplacée uniquement par LEU & ILE
Oligo 12 : GAACAGCGCC TTGAARA**K**GC CCTCGCCAAA CCC
Après criblage avec la 2-cyano-3-cyclopropyl-1-(2-méthylsulfonyl-4-trifluorométhylhényl) propan-1,3-dionede plusieurs centaines de mutants potentiels, on a identifié 15 nouveaux mutants, dont 12 simples mutants et 3 doubles mutants (Voir tableau récapitulatif), présentant une tolérance à l'inhibiteur 2-cyano-3-cyclopropyl-1-(2-méthylsulfonyl-4-trifluorométhylphényl) propan-1,3-dione dans notre test de détection colorimétrique. Il faut ajouter que certains des mutants identifiés au cours de ces mutagenèses dirigées sont identiques bien sûr aux mutants identifiés dans l'exemple 2 (comme par exemple PfS334 et de PfD334).

Il a été possible de combiner des mutations, non seulement dans des régions proches, comme c'est le cas pour obtenir des double mutants en position 334 et 336, mais aussi dans des régions éloignées comme c'est le cas pour obtenir des double mutants en position 215 et 336, comme indiqué ci-après.

pRP C PfL215 Delta : L'ADN plasmidique du clone pRP C PfL21 a été digéré PstI, purifié puis religué. Le premier site PstI se situant au niveau de la Leucine 289,et le dernier site PstI se situant dans le site de clonage multiple du PKK233.2, le clone obtenu pRPC PfL215 Delta, contient la partie de la séquence codante de l'HPPD Pf L215 incluant la mutation Pro215Leu, mais pas la partie terminale ni la partie génomique.
PRP C PfL215 W336. PRP C PfL215 E336, PRP C PfL215 I336 :

Les ADN plasmidiques des clones pRP CPfW336, pRP CPfE336, pRP CPfI336, ont été digéré par MIuI et HindIII, MluI se situant dans la partie codante de l'HPPD de Pseudomonas fluorescens, au niveau de l'acide aspartique 254, HindIII se situant dans le site de clonage multiple du PKK223.3. Les fragments contenant les parties C-terminales des HPPD mutées ainsi que la partie génomique, ont été purifiées, puis liguées dans le plasmide pRP C PfL215 Delta, digéré par MluI et HindIII. Les trois clones ainsi obtenus sont équivalents au clone pRP C PfL215, mais contiennent respectivement aussi la mutation W336, E336 et 1336.

Les mutants obtenus sont identifiés comme suit:

### Simples mutants:

| | |
|---|---|
| Phenylalanine333 remplacé par tryptophane | appelé PfW333 |
| Phenylalanine333 remplacé par leucine | appelé PfL333 |
| Glycine334 remplacé par tryptophane | appelé PfW334 |
| Glycine334 remplacé par proline | appelé PfP334 |
| Glycine334 remplacé par leucine | appelé PfL334 |
| Glycine334 remplacé par Isoleucine | appelé PfI334 |
| Glycine336 remplacé par acide aspartique | appelé PfD336 |
| Glycine336 remplacé par glutamine | appelé PfQ336 |
| Glycine336 remplacé par acide glutarique | appelé PfE336 |
| Glycine336 remplacé par tryptophane | appelé PfW336 |
| Glycine336 remplacé par isoleucine | appelé PfI336 |
| Asparagine337 remplacé par leucine | appelé PfL337 |

### Doubles mutants obtenus par mutagenèse dirigée:

| | |
|---|---|
| Glycine334 remplacé par alanine et | |
| Glycine336 remplacé par alanine | appelé PfA334-A336 |
| Glycine334 remplacé par alanine et | |
| Glycine336 remplacé par arginine | appelé PfA334-R336 |
| Glycine334 remplacé par serine et | |
| Glycine336 remplacé par arginine | appelé PfS334-R336 |

### Doubles mutants obtenus par clonage:

| | |
|---|---|
| Proline 215 remplacé par Leucine et | |
| Glycine 336 remplacé par Tryptophane | appelé PfL215-W336 |
| Proline 215 remplacé par Leucine et | |
| Glycine 336 remplacé par Acide Glutamique | appelé PtL215-E336 |
| Proline 215 remplacé par Leucine et | |
| Glycine 336 remplacé par Isoleucine | appelé PfL215-I336 |

Ce résultat montre qu'il est possible, par mutagenèse des acides aminés conservés entre les séquences protéiques de différentes HPPD situés dans la partie C-terminale de la protéine, d'obtenir des HPPD présentant une tolérance aux inhibiteurs d'activité HPPD. Donc toute région conservée entre différentes séquences en acides aminés d'HPPD est une bonne cible pour obtenir des mutants qu'il est intéressant d'analyser pour déterminer leur tolérance. Il est clair que fait partie de l'invention toute mutation ou mutation multiple qui permettrait d'obtenir une HPPD tolérante même si cette protéine n'est pas exemplifiée dans ce texte.

Il est là aussi démontré que le domaine C-terminal est bien la cible privilégiée de la mutagenèse d'une HPPD en vue d'une bonne tolérance de l'enzyme à ces différents inhibiteurs. En effet dans le domaine N-terminal défini comme allant de l'acide aminé n° 1 au peptide de liaison défini précédemment chez *Pseudomonas fluorescens* A32 ou à ces correspondants dans les HPPD d'autres espèces il est très difficile de définir une région conservée.

La ou les modifications, par mutagenèse dirigée à partir d'informations déduites d'un alignement de séquence, peuvent être faites sur toute protéine ayant une activité de type HPPD c'est à dire transformant le 4-hydroxyphénylpyruvate en homogentisate, dont la région codante est ou pourrait être clonée. Les HPPD décrites dans ce texte sont entre autres celle de *P. fluorescens, Arabidopsis thaliana, Daucus carola, Zea mais et Synechocystis,* mais bien sûr il est évident que toutes ces modifications sont applicables aux autres HPPD.

### Exemple 4: Mutagenèse dirigée du gène de l'HPPD de Pseudomonas fluorescens A32.

Les résultats obtenus par mutagenèse au hasard comme décrit à l'exemple 2, ainsi que de ceux obtenue après alignement des déférentes séquences montrent clairement que le peptide allant de F#333 à F#338 (numérotation sur HPPD de *P*. *fluorescens)* est une région particulièrement intéressante en terme de mutagenèse pour obtenir une tolérance.

Parallèlement à cette information, on a découvert, par une analyse de la structure tridimensionnelle de l'enzyme de *Pseudomonas fluorescens* strain A32 par exemple (figure 1), que la partie C-terminale de la protéine, de son monomère contient le site catalytique de l'enzyme. Ce sont donc les acides aminés de ce domaine C-terminal qu'il faut mutagénéiser en priorité pour espérer modifier l'interaction protéine inhibiteur et obtenir ainsi une enzyme plus tolérante.

On a donc recherché, par analyse de la structure tridimensionnelle de la protéine HPPD de *P. fluorescens,* des mutations ponctuelles qui pourraient entraîner un changement conformationnel de l'hexapeptide Phe333 à Phe338 sans muter directement ce peptide. L'idée directrice est que la mutation d'un acide aminé assez éloigné du site actif, un acide aminé non conservé peut influencer le positionnement dans l'espace de ce peptide de Phe333 à Phe338 et donc induire une tolérance aux inhibiteurs de l'HPPD par effet "ricochet".

On a donc recherché dans la structure des acides aminés proches de cet hexapeptide; un petit nombre d'acides aminés répondent à ce critère, comme l'acide aspartique 287 et la glycine 298 [pour information, l'atome de carbone alpha de la glycine 334 de l'hexapeptide est à 9,61 angström de celui du fer situé dans le site catalytique et le carbone alpha la glycine 298 est à 5,75 angström de celui de la glycine 334]. Cette glycine 298 a paru être des deux acides aminés le meilleur candidat car toute mutagenèse va conduire à un acide aminé avec une chaîne latérale plus grosse et on a remplacé cette glycine par un acide glutamique (dont la chaîne latérale est en outre chargée négativement) en vue d'une modification suffisamment sensible pour que l'effet soit visible.

De plus quand on mute la glycine 298 par un acide glutamique, la chaîne latérale de cet acide glutamique qui est très encombrée 'cogne' sur la structure secondaire en feuillet Beta dans laquelle on trouve le motif conservé LLQIF. La phénylalanine 312 de ce motif se trouve elle même très proche du fer.

On a testé la mutation Gly298 en Glu298.

Cette mutations Gly298 en Glu298 a été créée par mutagenèse dirigée (U.S.E. Mutagenesis Kit, Pharmacia) du vecteur pRP C en utilisant l'oligonucléotide n° 13 (identificateur de séquence en annexe - SEQ ID NO 13) :

### GLY298 remplacé par acide glutamique

Oligo 13 : GCCTTCCACGGAAGATTCGTCCAGCAGGATACC

Ce mutant appelé PfE298 provoque le brunissement du milieu de criblage contenant 7mM de RPA202248 (Voir tableau récapitulatif).

Ceci confirme qu'avec la connaissance de la structure tridimensionelle de l'HPPD, on peut déterminer un certain nombre de mutations efficaces en terme de tolérance aux inhibiteurs de l'HPPD, qu'elle soit d'origine bactérienne végétale ou autre. En effet il est tout à fait possible de modéliser la structure de n'importe quelle HPPD dont on a la séquence protéique puisque on connaît la structure précise dans le cas de l'HPPD de *P*. *fluorescens.* Cette modélisation sera surtout intéressante dans le cas de la modélisation du domaine C-terminale, le mieux conservé en terme de séquence primaire et surtout le plus prometteur en terme de tolérance aux inhibiteurs de l'HPPD.

La ou les modifications, par mutagenèse dirigée à partir d'informations déduites de la structure tridimensionelle d'une HPPD modélisée ou déterminée par analyse de cristaux obtenus en présence ou en abscence d'un inhibiteur, peuvent être faites sur toute protéine ayant une activité de type HPPD c'est à dire transformant le 4-hydroxyphénylpyruvate en homogentisate, dont la région codante est ou peut être clonée. Les HPPD décrites dans la présente demande sont entre autres celle de *P. fluorescens, Arabidopsis thaliana, Daucus carota, Zea mais et Synechocystis,* mais bien sûr il est évident pour l'homme de métier que toutes ces modifications sont applicables aux autres HPPD.

### Exemple 5: Expression d'HPPD mutantes de Pseudomonas fluorescens dans le tabac

### A) Construction de gènes chimères.

pRP-VB3: L'ADN du vecteur binaire pBI121 (Clontech) a été digéré HindIII et XbaI, les extrémités complétées avec des dNTP à l'aide de la pfu polymérase (Stratagène), le vecteur purifié. L' ADN du clone pRP-S décrit à l'exemple 2 de la demande dans la demande PCT 96/38567 et comprenant l'enchaînement " promoteur double histone - TEV - OTP - gène HPPD - terminateur Nos" a été digéré HindIII et SacI, les extrémités complétées avec des dNTP à l'aide de la pfu polymérase (Stratagène), l'insert purifié puis ligué dans le vecteur purifié précédemment décrit. L'orientation a été vérifié par digestion SalI. Le clone pRP-VB3 a donc la structure de gène chimérique suivante :

| |
|---|
| RB / promoteur Nos / NPTII / terminateur Nos / promoteur double histone / tev / otp /HPPD tronquée / terminateur Nos / LB |

RB LB = bordure droite et gauche respectivement du T-DNA *d'Agrobacterium tumefaciens*
promoteur Nos = promoteur de la nopaline synthase *d'Agrobacterium tumefaciens*
NPTII = séquence codante de la néomycine phosphotransferase de type II (conférant la résistance à la kanamycine)
terminateur NOS = séquence terminatrice de la nopaline synthase *d'Agrobacterium tumefaciens*
promoteur double histone = décrit dans EP 507 689
tev = TEV enhancer (Carrington & Freed)
otp = peptide de transit optimisé (EP 508 909)

pRP-VB3-b: L'ADN du clone pRP-VB3 a été digéré par BamHI purifié, puis ligué dans le vecteur pZERO-1 (Invitrogen) qui ne contient pas les sites de restriction BstEII et SalI, digéré BamHI et purifié. Une partie de l'OTP, le gène de l'HPPD et le terminateur Nos sont ainsi transférés dans pZERO-1.

pRP-VB3-c : L'ADN du clone pRPVB3-b a été digéré SalI, purifié, puis ligué en présence de l'adaptateur présenté ci-dessous (oligonucléotides 14 et 15 - SEQ ID NO 14 et 15 en annexe), de façon à remplacer le site de restriction SalI par le site BstEII.
5' TCGAGAGAGAGGTGACCGAGAGA 3'
3' CTCTCTCCACTGGCTCTCTAGCT 5'

pRP-VB3-d : L'ADN du clone pRP-VB3-c a été digéré BamHI, l'insert purifié, puis cloné dans le vecteur PUC19 (Biolabs) digéré BamHI. Une partie de l'OTP, le gène de l'HPPD et le terminateur Nos sont ainsi transférés dans PUC19.

pRP-VB3-e : Le vecteur PUC 19 ne possédant pas les sites de restriction PmlI et StuI, l'ADN du clone pRP-VB3-d a été digéré PmlI et StuI, purifié, puis ligué sur lui-même permettant ainsi de supprimer le site Not dans le gène de l'HPPD et de tronquer la partie codante de l'HPPD de 500 paires de bases environ, afin de rendre aisé par la suite, le criblage des colonies transformées ayant intégré les HPPD mutantes.

pRP-VB3-f : Le vecteur pRP-VB3-e a été digéré NotI, les extrémités complétées avec des dNTP à l'aide de la Pfu polymérase (Stratagène), purifié, digéré BamHI, purifié, puis cloné dans le vecteur PUC 19 digéré KpnI, les extrémités complétées avec des dNTP à l'aide de la Pfu polymérase (Stratagène), purifié, digéré BamHI, purifié.

pRP-VB83-g : L'ADN du clone pRP-VB3 à été digéré BstEII, les extrémités complétées avec des dNTP à l'aide de la Pfu polymérase (Stratagène), le vecteur purifié puis ligué sur lui-même permettant ainsi la disparition du site unique BstEII de ce vecteur.

gRP-RD224 : L'ADN du clone pRP-VB3-f a été digéré BamHI et SacI, purifié, puis ligué dans le vecteur pRP-VB3-g digéré par BamHI et SacI et purifié. Le clone pRP-RD224 a donc la structure suivante :

| |
|---|
| RB / promoteur Nos / NPTII / terminateur Nos / promoteur double histone / tev / otp /HPPD tronquée / terminateur Nos / LB |

pRP-RD224mutants : Les ADN des vecteurs portant les HPPD mutées ainsi que l'HPPD non mutée contenues dans le vecteur PKK233-2, ont été digérés par KpnI et BstEII, purifiées puis liguées dans le vecteur pRP-RD224, digéré par KpnI et BstEII et purifié. Les transformants ayant intégré le gène de l'HPPD muté ont été sélectionné pour la taille de l'insert par digestion KpnI et BstEII. Les clones obtenus sont notés pRP-RD224 auquel on ajoute le type de mutation effectuée sur l'HPPD, on a ainsi créé par exemple pRP RD224 Pf (pour l'enzyme non mutée) pRP RD224 PfD336 (pour l'enzyme avec un acide aspartique en 336),pRP RD224 PfQ336 (pour l'enzyme avec une glutamine en 336), pRP RD224 PfL333 (pour l'enzyme avec une leucine en 333), pRP RD224 PfA334~A336 (pour l'enzyme avec une alanine en 334 et en 336, double mutant).

### B) Transformation du Tabac "Petit havana".

Les gènes chimères décrits précédemmentont été transférés dans du tabac "Petit havana" selon les procédures de transformation et de régénération déjà décrites dans la demande européenne EP n° 0 508 909.

### 1) Transformation:

Le vecteur est introduit dans la souche non oncogène *d'Agrobacterium tumefaciens* EHA101.

### 2) Régénération:

La régénération du tabac "Petit havana" à partir d'explants foliaires est réalisée sur un milieu de base Murashige et Skoog (MS) comprenant 30g/l de saccharose ainsi que 350mg/l de céfotaxime et des doses variables de la 2-cyano-1-[4-(methylsulphonyl)-2-trifluoromethylphenyl]-3-(1-methylcyclopropyl)propan-1,3-dione(EP 496630), 10ppm, 20ppm, 40ppm qui est un analogue de 2-cyano-3-cyclopropyl-1-(2-méthylsulfonyl-4-trifluorométhylphényl) propan-1,3-dione ou directement des doses de 2 ou 4 ou 8 ppm de 2-cyano-3-cyclopropyl-1-(2-méthylsulfonyl-4-trifluorométhylphényl) propan-1,3-dione . Les explants foliaires sont prélevés sur des plants en serre et transformés selon la technique des disques foliaires (Science 1985, Vol 227, p1229-1231) en trois étapes successives :
- la première comprend l'induction des pousses sur un milieu MS additionné de 30g/l de saccharose contenant 0.05mg/l d'acide naphtylacétique (ANA). et 2mg/l de benzylaminopurine (BAP) pendant 15 jours et des doses variables d'herbicide, la 2-cyano-1-[4-(methylsulphonyl)-2-trifluoromethylphenyl]-3-(1-methylcyclopropyl)propan-1,3-dione(EP 496630) ou la 2-cyano-3-cyclopropyl-1-(2-méthylsulfonyl-4-trifluorométhyl phényl) propan-1,3-dione).
- Les pousses vertes formées au cours de cette étape sont ensuite développées par culture sur un milieu MS additionné de 30g/l de saccharose et des doses variables d'herbicide , mais ne contenant pas d'hormone, pendant 10 jours.
- Puis on prélève des pousses développées et on les cultive sur un milieu d'enracinement MS à teneur moitié en sels, vitamines et sucres et des doses variables d'isoxaflutole et ne contenant pas d'hormone. Au bout d'environ 15 jours, les pousses enracinées sont passées en terre.

### C) Mesure de la tolérance à l'herbicide des plantules in- vitro.

Les expériences sont réalisées par action d'un agent de sélection avec les mutants indiqués dans le tableau suivant et montrent que si l'on n'obtient pas de pousses/plantules aux doses les plus élevées d'herbicide dans les essais de transformation/régénération avec une HPPD non mutée, par contre les mutants permettent grâce à une meilleure tolérance de l'enzyme d'obtenir des plantules même à des doses élevées de 2-cyano-1-[4-(methylsulphonyl)-2-trifluoromethylphenyl]-3-(1-methylcyclopropyl)propan-1,3-dione(EP 496630) ou de 2-cyano-3-cyclopropyl-1-(2-méthylsulfonyl-4-trifluoro méthylphényl) propan-1,3-dione des plantules (voir tableau récapitulatif). Ces plantules obtenues à fortes concentration de l'agent de sélection avec une HPPD mutée et que l'on ne peut obtenir avec l'HPPD sauvage sont tout à fait normales.

Au cours de la sélection in-vitro sur 2-cyano-3-cyclopropyl-1-(2-méthylsulfonyl-4-trifluorométhylphényl) propan-1,3-dione à 1ppm, 2ppm, 4ppm et 8ppm, il apparaît qu'un certain nombre de mutants ne permettent pas la régénération aisée de plantules de tabac transformées, quelque soit la dose d'herbicide employée. On notera toutefois que des transformants pourront être obtenus avec un plus grand nombre d'essais de transformation, et/ou en déterminant les concentrations d'agent de sélection.

Par contre avec d'autres mutants il est aisé d'obtenir des plantules vertes et donc tout à fait normales. Les mutants qui donnent le plus de plantules sont les mutants PfL215, PfD336 et PfQ336 (pour les mutants étudiés selon ce procédé).

Au cours de la transformation avec PfD336,
il a été obtenu 25% des événements de transformation tolérant la sélection faite à 1ppm,
il a été obtenu 25% des événements de transformation tolérant la sélection faite à 2ppm,
il a été obtenu 35% des événements de transformation tolérant la sélection faite à 4ppm,
il a été obtenu 15% des événements de transformation tolérant la sélection faite à 8ppm.

Au cours de la transformation avec PfL215,
il a été obtenu 8% des événements de transformation tolérant la sélection faite à 1ppm,
il a été obtenu 60% des événements de transformation tolérant la sélection faite à 2ppm,
il a été obtenu 8% des événements de transformation tolérant la sélection faite à 4ppm,
il a été obtenu 24% des événements de transformation tolérant la sélection faite à 8ppm.

Au cours de la transformation avec PfQ336,
il a été obtenu 35% des événements de transformation tolérant la sélection faite à 1ppm,
il a été obtenu 25% des événements de transformation tolérant la sélection faite à 2ppm,
il a été obtenu 40% des événements de transformation tolérant la sélection faite à 4ppm,
il a été obtenu 0% des événements de transformation tolérant la sélection faite à 8ppm.

Au cours de la transformation avec l'HPPD non mutée,
il a été obtenu 15% des événements de transformation tolérant la sélection faite à 1ppm,
il a été obtenu 70% des événements de transformation tolérant la sélection faite à 2ppm,
il a été obtenu 15% des événements de transformation tolérant la sélection face à 4ppm,
il a été obtenu 0% des événements de transformation tolérant la sélection faite à 8ppm.

Ces résultats viennent donc confirmer que les HPPD mutées sont statistiquement plus efficaces que l'HPPD sauvage; avec les deux mutants PfD336 et PfL215 il est possible d'obtenir des transformants à la dose la plus haute de 2-cyano-3-cyclopropyl-1-(2-méthylsulfonyl-4-trifluorométhylphényl) propan-1,3-dione, alors que ce n'est pas possible avec l'HPPD non mutée.

De plus avec le mutant PfQ336, de nombreux événements de transformation sont obtenus à 4ppm de 2-cyano-3-cyclopropyl-1-(2-méthylsulfonyl-4-trifluorométhylphényl) propan-1,3-dione, 40% des plantules, alors qu'avec l'HPPD sauvage un faible pourcentage des plantes sont obtenus à 4ppM de cet herbicide.

**Tableau récapitulatif des mutants de l'HPPD de P. fluorescens**

| **Type d'HPPD** | **Activité dans test de criblage * avec une dose d'agent de sélection de** | | **% de plantules vertes obtenues à la dose de x ppm d'herbicide** |
|---|---|---|---|
| | **0 mM** | **7mM** | |
| **Sauvage** | 10 | 0 | 15% à 4ppm |
| **Mutée** | | | |
| PfL215 | 10 | 7 | 24% à 8ppm |
| PfE298 | 8 | 3 | pas de plantules vertes |
| PfL333 | 5 | 4 | - |
| PfW333 | - | - | pas de plantules vertes |
| Pf5334 | 7 | 7 | pas de plantules vertes |
| PfD334 | 1 | 1 | - |
| PfW334 | 6 | 6 | - |
| PfP334 | 5 | 5 | pas de plantules vertes |
| PfL334 | 5 | 4 | pas de plantules vertes |
| Pf1334 | 5 | 4 | pas de plantules vertes |
| PfS336 | - | - | - |
| PfD336 | 2 | 1 | 15% à 8ppm |
| PfQ336 | 8 | 8 | 40% à 8ppm |
| PfE336 | 10 | 8 | - |
| PfW336 | 10 | 9 | - |
| PfI336 | 10 | 8 | - |
| PfL337 | 8 | 8 | pas de plantules vertes |
| PfA334-A336 | 8 | 8 | - |
| PfA334-R336 | 8 | 7 | pas de plantules vertes |
| PfS334-R336 | 5 | 5 | pas de plantules vertes |

| | | | |
|---|---|---|---|
| * : test de criblage décrit dans l'exemple 1. '-' données non disponibles. | | | |

Le chiffre 10 correspond à une activité forte de même niveau que l'HPPD non mutée et sans inhibiteur, le chiffre 0 correspond à une activité nulle soit due à une inhibition complète par le 2-cyano-3-cyclopropyl-1-(2-méthylsulfonyl-4-trifluorométhyl phényl) propan-1,3-dione soit due à une mutation entraînant la non activité de l'enzyme, les chiffres là 9 correspondent à des activités intermédiaires plus forte quand le chiffre augmente dans le test d'évaluation décrit dans l'exemple 1.

Ces résultats confirment que l'introduction d'une HPPD présentant une tolérance aux inhibiteurs d'activité HPPD dans les plantes, confère à celles-ci une tolérance à ces même inhibiteurs, tolérance qui est donc meilleure que celle obtenue avec une HPPD non mutée.

### D) Tolérance in- vivo.

Toutes les plantes, après enracinement, ont été acclimatées à la serre et menées à graines.

Les graines de plusieurs capsules sont mélangées , puis semées sur un mélange de terreau et de sable, à raison de 100 graines environ par barquette de semis. Le génotype sauvage Petit Havana est inclus comme témoin. Les traitements sont effectués à raison de 600g/ha d'isoxaflutole formulé. Les notations d'aspect du semis sont réalisées 12 jours après traitement, la note 5 pour le meilleur aspect, la note 1 pour le plus mauvais. Les moyennes des observations de plusieurs événements de transformation (10-20 événements de transformation par HPPD évaluée) sont représentatives des valeurs de tolérance en pré émergence de l'HPPD sauvage et des HPPD mutées.

Ces mesures effectuées pour plusieurs mutants d'HPPD de *Pseudomonas fluorescens* sur les positions 336 et 215 permettent d'établir les valeurs moyennes de tolérance dans le tableau ci-après.

| **Essai** | **1** | **2** |
|---|---|---|
| **Pf non mutée** | 2,06 | 2,2 |
| **PfW336** | - | 2,8 |
| **PfD336** | 3,35 | - |
| **PfQ336** | 2,94 | - |
| **PfL215** | 3 | - |

Ces résultats montrent que les HPPD mutées selon l'invention présentent dans les plantes transformées une tolérance aux herbicides inhibiteurs d'HPPD supérieure à la tolérance obtenue avec l'HPPD native correspondante.

### Exemple 6 : Mutagenèse dirigée du gène de l'HPPD de Synechocystis.

On choisit un site de mutation efficace sur l'HPPD de *Pseudomonas fluorescens* en terme de tolérance aux inhibiteurs de l'HPPD et on le transpose à une autre HPPD.

Cette transposition est faite à une HPPD la plus divergente possible de l'HPPD de *Pseudomonas fluorescens* et des autres HPPD connues. Pour ce faire on travaille avec le gène codant pour l'HPPD de *Synechocystis* connue grâce au séquençage systématique du génome de cette cyanobactérie jamais cloné en tant que tel. On a donc d'abord isolé le gène puis on l'a fait exprimer dans *E. coli .*

### 1) obtention du gène.

L'ADN génomique de la cyanobactérie *Synechocystis* PCC6803 a été extrait et purifié en utilisant les protocoles standards. 200µg de cet ADN génomique ont été amplifiés par réaction de polymérisation en chaine (PCR) en utilisant 1,25U de pow polymérase (Böhringer) dans son tampon dans un volume réactionnel de 50 µl avec 200µM de dNTP(désoxyribonucleotide-triphosphate). Les séquences des oligonucléotides synthétiques 16 et 17 (SEQ ID NO 16 et 17 en annexe) utilisés comme amorces ont été déduites de la séquence publiée dans Genebank de l'HPPD de *Synechocystis.*
Oligo 16 ATTATGGAAT TCGACTATCT T
Oligo 17 CAGTATTCAT AATGTTAATT ATG

Le programme d'amplification, 5 minutes 94°C, puis 50 cycles 1 minute 94°C, 1 minute 49°C, 1 minute 72°C, puis 5 minutes 72°C, a été réalisé sur un appareil Perkin-Elmer 9600.

### 2) Clonage du gène et expression.

Le fragment amplifié obtenu par PCR a été purifié, digéré EcoRI, repurifié, puis cloné dans le vecteur ptrc-99A (Pharmacia) préalablement digéré EcoRI et SmaI. La bactérie JM105 a été transformée par le plasmide recombinant. Le séquencage a permis de vérifier la conformité du fragment cloné avec la séquence de l'HPPD de *Synechocystis* publiée.

L'activité dioxygénase de l'HPPD de *Synechocystis* ainsi obtenue a été observée par brunissement du milieu en utilisant le test colorimétrique décrit précédemment (cf exemple 1) avec ajout d'IPTG(isopropyl-β-D-thio-galactopyrazonide) à la concentration de 1mM afin d'induire l'expression de la protéine. Dans les mêmes conditions, sur un milieu contenant 7mM de 2-cyano-3-cyclopropyl-1-(2-méthylsulfonyl-4-trifluorométhylphényl) propan-1,3-dione, il n'y pas brunissement du milieu, ce qui confirme l'inhibition de l'activité HPPD de *Synechocystis* par le 2-cyano-3-cyclopropyl-1-(2-méthylsulfonyl-4-trifluorométhylphényl) propan-1,3-dione .

### 3) Mutagenèse dirigée.

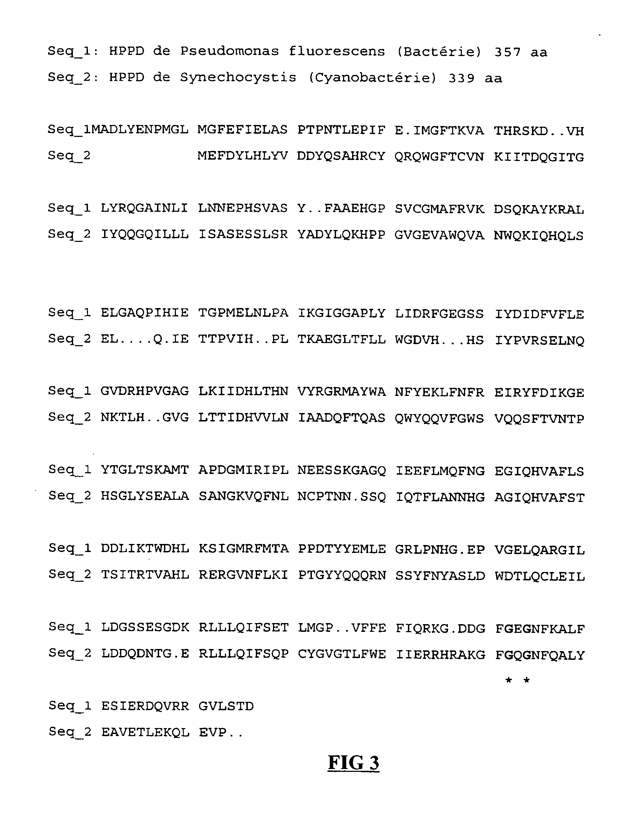

Par alignement des séquences protéiques de l'HPPD de *Pseudomonas fluorescens* A32 et de l'HPPD de *Synechocystis* PCC6803, on a estimé que les Glycines en position 334 et 336 de l'HPPD de *Pseudomonas* (glycines indiquées par des étoiles dans la figure de l'exemple 3 et qui sont très fortement conservées) sont en position 318 et 320 sur l'HPPD de *Synechocystis* (en gras sur l'alignement de séquences protéiques en figure 3)

Plusieurs mutants de la position 334 chez *Pseudomonas fluorescens* ayant été obtenus, deux expériences de mutagenèse dirigée (U.S.E., Pharmacia), utilisant les oligonucléotides MUGLYA et MUGLYB destinée à remplacer la Glycine 318 (de *Synechocystis* correspondant à la glycine 334 de *P. fluorescens)* soit en Asparagine ou en Sérine, soit en Proline ou Alanine, ont été réalisées (SEQ ID NO 18 et 19).
GLY318, remplacement possible par SER & ASN
Oligo 18 CGGGCAAAAG GATTTA**R**CCA AGG**AAA**CTTT CAAG
GLY318, remplacement possible par PRO & ALA
Oligo 19 CGGGCAAAAG GATTT**S**C**N**CA AGGAAACTTT CAAG

Dans les deux expériences, des clones obtenus après mutagenèse ont provoqué le brunissement du milieu de criblage à 7mM de 2-cyano-3-cyclopropyl-l-(2-méthylsulfonyl-4-trifluorométhylphényl) propan-1,3-dione (test décrit dans l'exemple 1). Un mutant de chaque expérience a été séquencé.

Glycine318 remplacé par asparagine, appelé SyN318 similaire au PfD334 (dans exemple 3)

Glycine318 remplacé par alanine, appelé SyA318 similaire au PfA334 (dans exemple 3)

Ces résultats confirment que les mutations conduisant à une tolérance, mise en évidence pour une HPPD donnée sont transposables à une autre HPPD, d'une autre espèce, d'un autre règne.

Ces résultats confirment aussi que des modifications de la séquence protéique dans la partie C-terminale d'une HPPD qu'elle que soit son origine (bactérienne ou autre) peuvent entraîner une tolérance améliorée aux inhibiteurs d'HPPD.

### Exemple7: Etude biochimique d'une HPPD mutée, mutants de Synechocystis.

Les mutants SyN318 et SyA318 obtenus dans l'exemple précédent ont été étudiés, en comparaison avec l'HPPD non mutée de *Synechocystis* pour les caractéristiques biochimiques Km et 150 vis à vis d'un inhibiteur de l'HPPD qui est le 2-cyano-3-cyclopropyl-1-(2-méthylsulfonyl-4-trifluorométhylphényl) propan-1,3-dione . Cette analyse a été faite en utilisant le protocole suivant:

### a) Mesure de l'activité

L'activité HPPD est mesurée en déterminant par chromatographie liquide de haute performance (HPLC) la quantité de produit formé, c'est à dire d'homogentisate, après incubation de l'enzyme avec son substrat, l'HPP. L'injection de différentes doses d'homogentisate variant de 12 à 48 nmoles dans la colonne permettent de déterminer le temps de rétention de l'homogentisate et de corrèler l'aire du pic avec la quantité de produit injecté.

Les mesures d'activité sont réalisées dans un volume final de 200 µl contenant : de l'ascorbate à 12,6 mM, du fer (FeH₈N₂O₈S₂,6H₂O) à 178 µM (préalablement préparé dans du tampon Tris acétate 0,1 M pH 6), 50 µg d'extrait brut contenant l'HPPD, de l'HPP à 352 µM, et du tampon Tris acétate 0,1 M pH 7,5. L'enzyme est incubée 1 min à 30°C avec dans un premier temps le fer puis 5 min à 30°C avec l'ascorbate avant le démarrage de la réaction par ajout du substrat, l'HPP. L'incubation est poursuivie durant 1 min 30 à 30°C, puis la réaction est arrêtée par addition de 70 µl d'acide perchlorique 20 %. Les protéines sont ensuite éliminées par centrifugation 5 min à 15300 rpm à 20°C. Le surnageant est récupéré. La quantité d'homogentisate formé est alors analysée par injection de 75 µl de l'essai dans une colonne Pico Tag C18 connecté au système HPLC. L'élution est réalisée à un débit de 1ml/min. L'élution isochratique réalisée est la suivante: 1- 0% de tampon B (soit 100% de tampon A : eau, acétonitrile 3% (v/v), acide trifluoro acétique 0, 1 % (v/v)) durant 6 minutes ; **2-** 100% de tampon B (acétonitrile 100%) jusqu'à la minute 11 ; **3-** 0% de tampon B jusqu'à la minute 19. En sortie de colonne, l'homogentisate est détecté par mesure de l'absorbance à 288 nm. La quantité de produit formé est définie par l'aire du pic sur le chromatogramme.

### b) Détermination du K_{M}

Le K_{M} de l'HPPD pour l'HPP est déterminé en mesurant la vitesse initiale de la réaction avec différentes concentrations d'HPP. Les réactions sont réalisées dans les conditions décrites ci-dessus avec des concentrations d'HPP allant de 5,5 µM à 1400 µM.

### c) Détermination de l'IC₅₀

L'IC₅₀ est déterminée par mesure de la vitesse initiale de la réaction dans les conditions décrites ci-dessus, après incubation de l'enzyme 10 min à 30°C avec le fer, l'ascorbate, le substrat à 1056micromolaire et des concentrations d'inhibiteur variables. Les concentrations de 2-cyano-3-cyclopropyl-1-(2-méthylsulfonyl-4-trifluorométhylphényl) propan-1,3-dione utilisées varient de 10⁻¹⁰ à 10⁻⁴ M..

Les valeurs de Km estimées de l'enzyme pour son substrat c'est à dire le 4-hydroxyphénylpyruvate, et les valeurs de 150 estimées à activités comparables, sont reportées dans le Tableau ci-après :

| | **HPPD native** | **SyN318** | **SyA318** |
|---|---|---|---|
| **Km** | 60uM | 470uM | 320uM |
| **150** | 80nM | 80uM | 40uM |

Ces résultats confirment que les mutations réalisées dans la partie C-terminale de la protéine, si elles influent en diminuant l'affinité de l'enzyme pour le substrat (Km), influent encore plus fortement en diminuant l'affinité de l'enzyme pour l'inhibiteur (150). En effet le rapport 150 d'une HPPD mutante sur 150 de l'HPPD non mutante (qui est l'image de la perte d'affinité pour l'inhibiteur) est de 1000 et 500 pour SyN 318 et SyA 318 respectivement alors que le rapport Km d'une HPPD mutante sur Km de l'HPPD non mutante (qui est l'image de la perte d'affinité pour le substrat) est de 8 et 5 pour SyN 318 et SyA 318 respectivement. Ceci est bien l'exemplification que ces deux mutants ont une affinité un peu moindre pour le substrat de l'enzyme mais surtout une affinité très nettement plus faible pour les inhibiteurs de l'enzyme dont la 2-cyano-3-cyclopropyl-1-(2-méthylsulfonyl-4-trifluorométhylphényl) propan-1,3-dione .

Ces résultats confirment que le test de criblage colorimétrique décrit dans l'exemple 1 permet bien de détecter des HPPD mutées présentant une tolérance aux inhibiteurs d'activité HPPD. Ils valident l'outil qu'on utilise pour cribler et identifier des mutants de l'HPPD tolérants aux inhibiteurs de l'HPPD. Cet outil de screening est rapide et simple d'utilisation et c'est pourquoi on l'utilise mais il est clair que tout autre outil peut être utilisé pour faire une analyse similaire; il serait possible d'utiliser
- un outil de criblage mesurant l'activité et son inhibition basé sur la disparition d'HPP (dosage radioactif, dosage spectrophotométrique ou autre) ou basé sur la consommation d'oxygène ou basé sur l'apparition d'homogentisate (avec couplage d'une autre activité enzymatique)
- un outil de criblage vivo, comme par exemple la croissance de bactéries sur HPP comme seule source de carbone en présence d'inhibiteurs de l'HPPD permettant de sélectionner les seuls clones avec une HPPD tolérantes
- il est de même tout à fait envisageable d'utiliser un screening vivo dans les plantes en utilisant des vecteurs de transformation des plantes, des systèmes de transformation, des systèmes de régénération et de sélection avec un inhibiteur de l'HPPD comme ceux décrits dans l'exemple 5 et 8.

### Exemple 8: Evaluation de l'HPPD de Synechocystis non mutée et mutée, SyN318 et mutée SyA318, dans le tabac.

### A) Construction des gènes chimères:

Le vecteur utilisé pour faire la construction permettant l'expression de l'HPPD de *Synechocystis* (sauvage ou mutantes) dans les plantes de tabac de type PBD6 s'appelle pRD224 (décrit dans l'exemple 5). Ce vecteur a été initialement conçu pour cloner tous les mutants de l'HPPD de *Pseudomonas* par simple remplacement du gène de l'HPPD tronquée de ce vecteur au niveau des sites Kpn I et BstE II. Il peut également servir au clonage du gène de l'HPPD de *Synechocystis* en vue de la création d'une plante transgénique.

### B) Stratégie de clonage

La séquence codant pour l'HPPD de *Synechocystis* est clonée dans le vecteur pRD224 par remplacement de la séquence codant pour l'HPPD de *Pseudomonas fluorescens* tronquée. Cependant elle ne peut pas être clonée directement entre les sites Kpn I et BstE II, car les séquences N terminales des gènes d'HPPD de *Synechocystis* et de *Pseudomonas* sont très différentes. Mais le clonage entre le site Bam HI de l'OTP et le site BstE II est possible. Pour cela, il est nécessaire de recréer du côté 5', en amont de la séquence HPPD, le site Bam HI suivi de la partie codant pour l'OTP située en aval de Bam HI.

Le gène de l'HPPD de *Synechocystis* contenu dans le vecteur pTRC 99A est amplifié par réaction de polymérisation en chaîne (PCR) à l'aide des amorces A et B. L'oligonucléotide A permet d'ajouter le site Bam HI en amont du gène de l'HPPD ainsi qu'une partie de la séquence de l'OTP entre le site Bam HI et le début du gène. L'oligonucléotide B permet d'ajouter le site BstE II en aval du gène. Les amorces A et B sont indiquées dans les SEQ ID NO 20 et 21: 
oligonucléotideA: 5'NNNNNNNNNN GGATCCGGTG CATGGAATTC
GACTATCTTC3'
 oligonucleotideB: ^{5'}NNNNNNNNNN ***GGTCACC***AGT ATTCATAATG TTAATTATG^{3'}

La réaction d'amplification est réalisée à une température d'hybridation de 52°C. Les produits PCR sont ensuite séparés sur gel d'agarose. La bande d'ADN correspondant au gène HPPD est découpée puis purifiée.

Les fragments ainsi amplifiés sont digérés par Bam HI durant 1heure à 37°C puis par BstE II durant 1 heure à 60°C. Cet insert est ensuite isolé sur gel d'agarose. La bande d'ADN correspondant au gène HPPD est découpée puis purifiée.

Ce fragment est ensuite cloné dans le vecteur binaire. Celui-ci est au préalable digéré par Bam HI et BstE II, puis séparé du fragment correspondant à l'HPPD tronquée sur gel d'agarose. Ce vecteur est alors purifié de la même manière que le gène de l'HPPD.

La ligation entre le vecteur binaire et l'insert est réalisée sur la nuit à 16°C par la T4 DNA ligase. Le mélange de ligation est utilisé pour transformer des cellules d'*E.coli.* DH10B électrocompétentes. Les clones positifs sont sélectionnés sur du milieu LB contenant de la kanamycine à 50µg/ml. La présence de l'insert d'intérêt dans le vecteur binaire est contrôlée sur gel d'agarose après minipréparation et digestion de l'ADN plasmidique par Bam HI et Sac I durant 1 heure à 37°C. Le vecteur recombinant est alors utilisé pour transformer des cellules d'Agrobacterium tumefaciens EHA105 électrocompétentes. La sélection se fait sur milieu LB contenant de la kanamycine à 50µg/ml. Ce vecteur recombinant est donc porteur d'un TDNA contenant le gène de résistance à la kanamycine, et une séquence codante pour un ensemble OTP-HPPD Synechocystis sous le contrôle du promoteur double histone et d'un terminateur Nos.

### C) Transformation/Réneration du tabac variété PBD6

La transformation est réalisée comme cela est présentée dans l'exemple 5 sauf que la sélection est faite d'abord avec 200 mg/ml de kanamycine.

Par contre les jeunes pousses obtenues sur kanamycine sont excisées et transférées individuellement sur un milieu dépourvu d'hormones pour favoriser leur enracinement, et contenant du 2-cyano-3-cyclopropyl-1-(2-méthylsulfonyl-4-trifluorométhylphényl) propan-1,3-dione pour sélectionner les plantules transgéniques tolérantes à cet herbicide. Il s'agit du milieu MS (SIGMA M-5519 4,4 g/l) contenant 350 mg/l de cefotaxine, 1% saccharose (p/v), et 0 ou 8 ppm de 2-cyano-3-cyclopropyl-1-(2-méthylsulfonyl-4-trifluorométhylp hényl) propan-1,3-dione. La surproduction de l'HPPD dans les cellules transformées permet le développement de plantules chlorophylliennes tolérantes au 2-cyano-3-cyclopropyl-1-(2-méthylsulfonyl-4-trifluorométhylphényl) propan-1,3-dione , alors que les plantules issues de cellules non transformées, sensibles à l'herbicide, apparaissent totalement blanches.

Après deux semaines, les racines sont suffisamment développées pour que les plantules puissent être transférées en terre et cultivées en serre.

### D) Résultats

A partir de 60 disques foliaires en moyenne, environ 40 pousses sont régénérées pour chaque construction. Après 2 jours de culture sur milieu en présence de la 2-cyano-3-cyclopropyl-1-(2-méthylsulfonyl-4-trifluorométhylphényl) propan-1,3-dione, certaines plantules commencent à blanchir. Après 8 jours d'enracinement, ce blanchissement est assez significatif pour être interprétable. A 8 ppm de 2-cyano-3-cyclopropyl-1-(2-méthylsulfonyl-4-trifluorométhylphényl) propan-1,3-dione, on constate que seules 40% des plantes contenant l'enzyme sauvage survivent, contre 72% pour les plantes contenant l'enzyme SyA318 et 88% pour celle contenant l'enzyme SyN318. Les plantes contenant les enzymes mutées présentent donc une meilleure tolérance que celles contenant l'enzyme sauvage.

Après plus d'un mois sur 8 ppm de 2-cyano-3-cyclopropyl-1-(2-méthylsulfonyl-4-trifluorométhylphényl) propan-1,3-dione , le nombre de plantules vertes pour la transformation avec l'HPPD sauvage est de 0%, pour SyN318 ce pourcentage est de 17 % et pour le mutant SyA318 il est de 19%.

Parallèlement si la régénération est faite sur la 2-cyano-1-[4-(methylsulphonyl)-2-trifluoromethylphenyl]-3-(1-methylcyclopropyl)propan-1,3-dione(EP 496630), à des doses de 5 à 10 ppm (un inhibiteur moins fort des HPPD), les trois gènes permettent d'obtenir des plantes morphologiquement tout à fait conformes. Ceci confirme si besoin était que la surexpression d'une HPPD mutée ou non mutée permet d'obternir une tolérance au laboratoire.

Ces résultats semblent en accord avec les résultats obtenus *in vitro* par cinétiques enzymatiques et il semble bien possible d'établir une corrélation entre les mesures biochimiques *in vitro* et les résultats *in vivo.*

Ce dernier exemple confirme la cohérence au moins partielle entre screening in vitro (exemple 1), analyse biochimique (exemple 6) et tolérance d'une plante.

## Revendications

1. Hydroxy-phényl pyruvate dioxygénase (HPPD) mutée conservant ses propriétés dé catalyse du para-hydroxy-phenyl-pyruvate (HPP) en homogentisate ,moins sensible aux inhibiteurs d'HPPD que RHPPD d'origine non mutée, **caractérisée en ce qu'**été comprend au moins une mutation dans sa partie C-terminate, ladite partie C-terminate étant constituée par !a séquence protéique comprise entre d'une part le peptide de Saison et d'autre part l'extrémité C-terminate de l'enzyme.

2. HPPD mutée selon la revendication 1, **caractérisée en ce que** le peptide de liaisaon représentant l'extrémité N-terminale de la partie C-terminate de l'HPPD se situe entre 5 et 15 acides aminés en amont de l'acide aminé Asp161, par référence à LHPPD de *Pseudomonas.*

3. HPPD mutée selon l'une des revendications 1 ou 2, **caractérisée en ce que** la mutation est effectuée sur des acides aminés, qui sont remplacés par des acides aminés d'encombrement stérique supérieur.

4. HPPD mutée selon la revendication 3, **caractérisée en ce que** l'acide aminé de faible encombrement stérique est la glycine (Gly).

5. HPPO mutée selon l'une des revendications 1 à 4, **caractérisée en ce que** l'acide aminé du site de mutation est remplacé par l'un des acides aminés suivants: glutamine (Gln), acide glutamique (Glu), leucine (Leu), isoleucine (Ile) ou tryptophane (Trp).

6. HPPD mutée selon l'une des revendications 1 à 5, **caractérisée en ce que** la mutation est effectuée sur un acide aminé de la partie C-terminate commun à plusieurs séquences d'HPPD.

7. HPPD mutée selon l'une des revendications 1 à 6, **caractérisée en ce qu'**elle comprend dans sa partie C-terminale, la séquence peptidique suivante :
-Gly- Phe-Xaa-Yaa-Xab-Asn-Phe-Yab-Yac- Leu-Phe-+
dam laquelle
Xaa et Xab représentent indépendamment l'un de l'autre la glycine (Gly) ou un acide aminé d'encombrement supérieur à la glycine, étant entendu que si l'un de Xaa ou Xab représente Gly, alors l'autre est différent de Gly,
Yaa représente Ala, Lys ou Glu,
Yab représente Lys, ser, Arg ou Asn,et
Yac représente Ala, Ser, Glu ou Gln.

8. HPPD mutée selon la revendication 7, **caractérisée en ce que** l'un au moins de Xaa ou Xab représente Leu, Glu, Trp ou Ile.

9. HPPD mutée selon la revendication 8, **caractérisé**é en ce que Xab représente Glu, Trp ou Ile, de préférence Trp.

10. HPPD mutée selon l'une des revendications à 9, **caractérisée en ce que** par référence à la séquence d'HPPD de *Pseudomanas,* les acides aminés mutés sont choisis parmi les acides aminés suivants: Pro215, Gly298, Gly332, Phe333, Gly334, Gly336 et Asn 337, préférentiellement les acides aminés Pro215 et Gly336.

11. HPPD mutée selon la revendication 10, **caractérisée en ce qu'**elle comprend une mutation choisie parmi les mutations suivantes : Pro215Leu, Gly336Glu, Gly338Trp ou Gly3361Ile.

12. Séquence d'acide nucléique codant pour une HPPD mutée conservant ses propriétés de catalyse de l'HPP en homogentisate selon l'une des revendications 1 à 11.

13. Gène chimère comprenant une séquence codante ainsi que des éléments de régulation en position 5' et, 3' hétérologues pouvant fonctionner dans un organisme hôte, en particulier les cellules végétales ou les plantes, **caractérisé en ce que** la séquence codante comprenant au moins une séquence d'acide nucléique- codant pour une HPPD mutée conservant ses propriétés de catalyse de l'HPP en homogentisate telle que définie précédemment.

14. Gène chimère selon la revendication 13, **caractérisé en ce que** l'organisme hôte est choisi parmi les bactéries, par exemple *E. coli*. les levures, en particulier des genres *Saccharomyces* ou *Kluyveromyces,Pichia,* les champignons, en particulier *Aspergillus,* les baculovirus, ou les cellules végétales et les plantes.

15. Gène chimère selon ta revendication 14, **caractérisée en ce que** l'organisme hôte est une cellule végétale ou une plante.

16. Gène chimère selon la revendication 15, **caractérisé en ce qu'**il comprend en 5' de la séquence d'acide nucléique- codant pour une HPPD mutée, une séquence d'acide nucléique codant pour un peptide de transit de plante cette séquence étant disposée entre la région promotrice et la séquence codant pour l'HPPD mutée de manière à permettre l'expression d'une protéine de fusion peptide de transition/HPPD mutée.

17. Protéine, de fusion peptide de transit/HPPD mutée, l'HPPD mutée étant définie selon rune des revendications 1 à 11.

18. Vecteur de clonage et/ou d'expression pour la transformation d'un organisme hôte **caractérisé en ce qu'**il contient au moins un gène chimère selon l'une des revendications 13 a 16.

19. Procédé de transformation d'un organismes hôte, **caractérisé en ce que** l'on intègre de manière stable dans ledit organisme hôte au moins une séquence d'acide nucléique selon la revendication 12 ou un gène chimère selon, rune des revendications 13 à 16.

20. Procédé selon la revendication 19, **caractérisé en ce que** l'organisme hôte est une cellule végétale.

21. Procédé selon ta revendication 20, **caractérisé en ce que** l'on régénère une plante à partir de la cellule végétale transformée.

22. Organisme hôte transformé en particulier cellule végétale ou plante, **caractérisé en ce qu'**il contient une séquence d'acide nucléique selon la revendication 12 ou un gène chimère selon rune des revendication 13 à 16.

23. Cellule végétale **caractérisé en ce qu'**il contient une séquence d'acide nucléique selon la revendication 12 ou un gène chimère selon l'une des revendication 13 à 16.

24. Plante transformée, **caractérisée en ce qu'**elle contient des cellules transformées selon la revendication 23.

25. Plante selon la revendication 24, **caractérisée en ce qu'**elle est régénérée à partir des cellules transformées selon la revendication 23.

26. Plante transformée **caractérisée en ce qu'**elle est issues de la culture et/ou du croisement des plantes régénérées selon la revendication 25.

27. Plantes transformées selon l'une des revendications 24 à 26, **caractérisées en ce qu'**elles sont choisies parmi les monocotylédones, notamment les céréales, la canne A sucre, le riz et le maïs, ou les dicotyledones, notamment le tabac, la soja, le colza, le coton, la betterave et le trèfle.

28. Graine transformée, **caractérisée en ce qu'**elle contient des cellules transformées selon la revendication 23 et qu'elle provient d'une plante transformée selon l'une des revendications 24 à 27.

29. Procédé de contrôle des mauvaises herbes dans une surface d'un champ comprenant des graines selon la revendication 28 ou des plantes transformées selon l'une des revendications 24 à 27, lequel procédé consiste à appliquer dans la dite surface du champ une dose toxique pour les dites mauvaises herbes d'un herbicide inhibiteur d'HPPD. sans toutefois affecter de manière substantielle les gaines ou plantes transformée avec le dit gène chimère selon l'invention.

30. Procédé de culture des plantes transformées selon l'une des revendications 24 à 27. lequel procédé consiste à planter les graines des dites plantes transformées selon la revendication 28 dans une surface d'un champ approprié pour la culture des dites plantes, a appliquer sur ta dite surface du dit champ une dose toxique pour les mauvaises herbes d'un herbicide ayant pour cible l'HPPD en cas de présence de mauvaises herbes, sans affecter de manière substantielle les dites graines ou les dites plantes transformées, puis à récolter les plantes cultivées lorsqu'elles arrivent à la maturité souhaitée et éventuellement à séparer les graines des plantes récoltées.

31. Procédé selon l'une des revendications 29 ou 30, **caractérisé en ce que** l'inhibiteur d'HPPD est choisi parmi les isoxazoles, en particulier l'isoxaflutole, les dicétonitriles, en particulier la 2-cyano-3-cycloprapyl-1-(2-SO₂ CH₃-4-CF₃ phényl) propane-1,3-dione et la 2-cyano-3-cyclopropyl-1-(2-SO₂ CH₃-4-2;3 Cl₂ phényl) propane-1,3-dione, les tricétones, en particulier la sulcotrione, ou les pyrazotinates.

## Claims

1. Mutated hydroxyphenylpyruvate dioxygenase (HPPD) which retains its properties of catalysing para-hydroxyphenylpyruvate (HPP) to homogentisate and which is less sensitive to HPPD inhibitors than is the original unmutated HPPD, **characterized in that** it contains at least one mutation in its C-terminal part, the said C-terminal part consisting of the protein sequence which is contained between the linking peptide, on the one hand, and the C-terminal end of the enzyme, on the other hand.

2. Mutated HPPD according to Claim 1, **characterized in that** the linking peptide representing the N-terminal end of the C-terminal part of the HPPD is located between 5 and 15 amino acids upstream of the amino acid Asp161, with reference to the *Pseudomonas* HPPD.

3. Mutated HPPD according to either of Claims 1 and 2, **characterized in that** the mutation is effected on amino acids which are replaced with amino acids which exhibit greater steric hindrance.

4. Mutated HPPD according to Claim 3, **characterized in that** the amino acid of low steric hindrance is glycine (Gly).

5. Mutated HPPD according to one of Claims 1 to 4, **characterized in that** the amino acid of the mutation site is replaced with one of the following amino acids: glutamine (Gln), glutamic acid (Glu), leucine (Leu), isoleucine (Ile) or tryptophan (Trp).

6. Mutated HPPD according to one of Claims 1 to 5, **characterized in that** the mutation is effected on an amino acid of the C-terminal part which is common to several HPPD sequences.

7. Mutated HPPD according to one of Claims 1 to 6, **characterized in that** it contains, in its C-terminal part, the following peptide sequence:
- Gly - Phe - Xaa - Yaa - Xab - Asn - Phe - Yab - Yac - Leu - Phe -
in which Xaa and Xab, independently of each other, represent glycine (Gly) or an amino acid which exhibits a hindrance which is greater than that of glycine, with it being understood that if either Xaa or Xab represents Gly, the other amino acid is then different from Gly,
Yaa represents Ala, Lys or Glu,
Yab represents Lys, Ser, Arg or Asn, and
Yac represents Ala, Ser, Glu or Gln.

8. Mutated HPPD according to Claim 7, **characterized in that** at least one of Xaa and Xab represents Leu, Glu, Trp or Ile.

9. Mutated HPPD according to Claim 8, **characterized in that** Xab represents Glu, Trp or Ile, preferably Trp.

10. Mutated HPPD according to one of Claims 1 to 9, **characterized in that**, with reference to the *Pseudomonas* HPPD sequence, the mutated amino acids are selected from the following amino acids: Pro215, Gly298, Gly332, Phe333, Gly334, Gly336 and Asn337, preferably the amino acids Pro215 and Gly336.

11. Mutated HPPD according to Claim 10, **characterized in that** it contains a mutation which is selected from the following mutations: Pro215Leu, Gly336Glu, Gly336Trp or Gly336Ile.

12. Nucleic acid sequence which encodes a mutated HPPD which retains its properties of catalysing HPP to homogentisate according to one of Claims 1 to 11.

13. Chimeric gene which comprises a coding sequence as well as heterologous regulatory elements, in the 5' and 3' positions, which are able to function in a host organism, in particular plant cells or plants, **characterized in that** the coding sequence contains at least one nucleic acid sequence which encodes a mutated HPPD which retains its properties of catalysing HPP to homogentisate as previously defined.

14. Chimeric gene according to Claim 13, **characterized in that** the host organism is selected from bacteria, for example *E. coli,* yeasts, in particular the genera *Saccharomyces* or *Kluyveromyces, Pichia,* fungi, in particular *Aspergillus,* baculoviruses or plant cells and plants.

15. Chimeric gene according to Claim 14, **characterized in that** the host organism is a plant cell or a plant.

16. Chimeric gene according to Claim 15, **characterized in that** it contains, in 5' of the nucleic acid sequence which encodes a mutated HPPD, a nucleic acid sequence which encodes a plant transit peptide, with this sequence being arranged between the promoter region and the sequence encoding the mutated HPPD so as to permit expression of a transit peptide/mutated HPPD fusion protein.

17. Transit peptide/mutated HPPD fusion protein, with the mutated HPPD being defined in accordance with one of Claims 1 to 11.

18. Cloning and/or expression vector for transforming a host organism, **characterized in that** it contains at least one chimeric gene according to one of Claims 13 to 16.

19. Method for transforming a host organism, **characterized in that** at least one nucleic acid sequence according to Claim 12 or one chimeric gene according to one of Claims 13 to 16 is stably integrated into the said host organism.

20. Method according to Claim 19, **characterized in that** the host organism is a plant cell.

21. Method according to Claim 20, **characterized in that** a plant is regenerated from the transformed plant cell.

22. Transformed host organism, in particular a plant cell or plant, **characterized in that** it contains a nucleic acid sequence according to Claim 12 or a chimeric gene according to one of Claims 13 to 16.

23. Plant cell, **characterized in that** it contains a nucleic acid sequence according to Claim 12 or a chimeric gene according to one of Claim 13 to 16.

24. Transformed plant, **characterized in that** it contains transformed cells according to Claim 23.

25. Plant according to Claim 24, **characterized in that** it is regenerated from the transformed cells according to Claim 23.

26. Transformed plant, **characterized in that** it is derived by cultivating and/or crossing the regenerated plants according to Claim 25.

27. Transformed plants according to one of Claims 24 to 26, **characterized in that** they are selected from monocotyledones, in particular cereals, sugar cane, rice and maize, or dicotyledones, in particular tobacco, soya bean, rape, cotton, beetroot and clover.

28. Transformed seed, **characterized in that** it contains transformed cells according to Claim 23 and **in that** it originates from a transformed plant according to one of Claims 24 to 27.

29. Method for controlling weeds in an area of a field which contains transformed seeds according to Claim 28 or plants according to one of Claims 24 to 27, which method consists in applying, to the said area of the field, a dose of an HPPD inhibitor herbicide which is toxic for the said weeds, without, however, significantly affecting the seeds or plants which have been transformed with the said chimeric gene according to the invention.

30. Method for cultivating the plants which have been transformed according to one of Claims 24 to 27, which method consists in planting the seeds of the said transformed plants according to Claim 28 in an area of a field which is appropriate for cultivating the said plants, in applying, if weeds are present, a dose, which is toxic for the weeds, of a herbicide whose target is the HPPD to the said area of the said field, without significantly affecting the said transformed seeds or the said transformed plants, and in then harvesting the cultivated plants when they reach the desired stage of maturity and, where appropriate, in separating off the seeds of the harvested plants.

31. Method according to either of Claims 29 and 30, **characterized in that** the HPPD inhibitor is selected from isoxazoles, in particular isoxaflutole, diketonitriles, in particular 2-cyano-3-cyclopropyl-1- (2-SO₂CH₃-4-CF₃phenyl) - propane-1,3-dione and 2-cyano-3-cyclopropyl-1-(2-SO₂CH₃-4-2,3 Cl₂ phenyl) propane-1, 3-dione, triketones, in particular sulcotrione, or pyrazolinates.

## Patentansprüche

1. Mutierte Hydroxyphenylpyruvatdioxygenase (HPPD), deren Eigenschaften bezüglich der Katalyse von para-Hydroxyphenylpyruvat (HPP) zu Homogentisat erhalten bleiben und die gegen HPPD-Hemmer weniger empfindlich ist als die nicht mutierte Ausgangs-HPPD, **dadurch gekennzeichnet, daß** sie mindestens eine Mutation in ihrem C-terminalen Abschnitt umfaßt, wobei dieser C-terminale Abschnitt aus der Proteinsequenz zwischen dem Bindungspeptid einerseits und dem C-terminalen Ende des Enzyms andererseits besteht.

2. Mutierte HPPD nach Anspruch 1, **dadurch gekennzeichnet, daß** das Bindungspeptid, das das N-terminale Ende des C-terminalen Abschnitts der HPPD darstellt, zwischen 5 und 15 Aminosäuren stromaufwärts der Aminosäure Asp161 in Bezug auf die HPPD von *Pseudomonas* liegt.

3. Mutierte HPPD nach Anspruch 1 oder 2, **dadurch gekennzeichnet, daß** die Mutation an Aminosäuren durchgeführt wird, die durch Aminosäuren mit einer größeren sterischen Raumfüllung ersetzt werden.

4. Mutierte HPPD nach Anspruch 3, **dadurch gekennzeichnet, daß** es sich bei der Aminosäure mit kleiner sterischer Raumfüllung um Glycin (Gly) handelt.

5. Mutierte HPPD nach einem der Ansprüche 1 bis 4, **dadurch gekennzeichnet, daß** die Aminosäure der Mutationsstelle durch eine der folgenden Aminosäuren ersetzt wird: Glutamin (Gln), Glutaminsäure (Glu), Leucin (Leu), Isoleucin (Ile) oder Tryptophan (Trp).

6. Mutierte HPPD nach einem der Ansprüche 1 bis 5, **dadurch gekennzeichnet, daß** die Mutation an einer Aminosäure des C-terminalen Abschnitts, die mehreren HPPD-Sequenzen gemeinsam ist, durchgeführt wird.

7. Mutierte HPPD nach einem der Ansprüche 1 bis 6, **dadurch gekennzeichnet, daß** sie in ihrem C-terminalen Abschnitt die folgende Peptidsequenz umfaßt:
- Gly - Phe - Xaa - Yaa - Xab - Asn - Phe - Yab - Yac - Leu - Phe -
in der
Xaa und Xab unabhängig voneinander Glycin (Gly) oder eine Aminosäure mit größerer Raumfüllung als Glycin bedeuten, wobei, wenn eine der Aminosäuren Xaa oder Xab Gly bedeutet, die andere nicht Gly bedeutet,
Yaa Ala, Lys oder Glu bedeutet,
Yab Lys, Ser, Arg oder Asn bedeutet, und
Yac Ala, Ser, Glu oder Gln bedeutet.

8. Mutierte HPPD nach Anspruch 7, **dadurch gekennzeichnet, daß** mindestens eine der Aminosäuren Xaa oder Xab Leu, Glu, Trp oder Ile bedeutet.

9. Mutierte HPPD nach Anspruch 8, **dadurch gekennzeichnet, daß** Xab Glu, Trp oder Ile, vorzugsweise Trp, bedeutet.

10. Mutierte HPPD nach einem der Ansprüche 1 bis 9, **dadurch gekennzeichnet, daß** die in Bezug auf die Sequenz der Pseudomonas-HPPD mutierten Aminosäuren aus der Reihe der folgenden Aminosäuren ausgewählt sind: Pro215, Gly298, Gly332, Phe333, Gly334, Gly336 und Asn337, vorzugsweise den Aminosäuren Pro215 und Gly 336.

11. Mutierte HPPD nach Anspruch 10, **dadurch gekennzeichnet, daß** sie eine Mutation aus der Reihe der folgenden Mutationen umfaßt: Pro215Leu, Gly336Glu, Gly336Trp oder Gly336Ile.

12. Nukleinsäuresequenz, die für eine mutierte HPPD, deren Eigenschaften bezüglich der Katalyse von HPP zu Homogentisat erhalten bleiben, nach einem der Ansprüche 1 bis 11 codiert.

13. Chimäres Gen, das eine Codiersequenz sowie heterologe Regulationselemente in 5'- und 3'-Position, die in einem Wirtsorganismus, insbesondere Pflanzenzellen oder Pflanzen funktionsfähig sind, umfaßt, **dadurch gekennzeichnet, daß** die Codiersequenz mindestens eine wie oben definierte Nukleinsäuresequenz, die für eine mutierte HPPD codiert, deren Eigenschaften bezüglich der Katalyse von HPP zu Homogentisat erhalten bleiben, umfaßt.

14. Chimäres Gen nach Anspruch 13, **dadurch gekennzeichnet, daß** der Wirtsorganismus aus der Reihe der Bakterien, zum Beispiel *E. coli,* Hefen, insbesondere den Gattungen *Saccharomyces oder Kluyveromyces, Pichia,* Pilze, insbesondere *Aspergillus,* Baculoviren oder Pflanzenzellen und Pflanzen stammt.

15. Chimäres Gen nach Anspruch 14, **dadurch gekennzeichnet, daß** es sich bei dem Wirtsorganismus um eine Pflanzenzelle oder eine Pflanze handelt.

16. Chimäres Gen nach Anspruch 15, **dadurch gekennzeichnet, daß** es in 5'-Position von der für eine mutierte HPPD codierenden Nukleinsäuresequenz eine Nukleinsäuresequenz, die für ein pflanzliches Transitpeptid codiert, umfaßt, wobei diese Sequenz zwischen der Promoterregion und der für die mutierte HPPD codierenden Sequenz liegt, sodaß die Expression eines Fusionsproteins Transitpeptid/mutierte HPPD ermöglicht wird.

17. Fusionspeptid Transitpeptid/mutierte HPPD, wobei die mutierte HPPD nach einem der Ansprüche 1 bis 11 definiert ist.

18. Klonierungs- und/oder Expressionsvektor für die Transformation eines Wirtsorganismus, **dadurch gekennzeichnet, daß** er mindestens ein chimäres Gen nach einem der Ansprüche 13 bis 16 enthält.

19. Verfahren zur Transformation eines Wirtsorganismus, **dadurch gekennzeichnet, daß** man mindestens eine Nukleinsäuresequenz nach Anspruch 12 oder ein chimäres Gen nach einem der Ansprüche 13 bis 16 stabil in den Wirtsorganismus integriert.

20. Verfahren nach Anspruch 19, **dadurch gekennzeichnet, daß** es sich bei dem Wirtsorganismus um eine Pflanzenzelle handelt.

21. Verfahren nach Anspruch 20, **dadurch gekennzeichnet, daß** man aus der transformierten Pflanzenzelle eine Pflanze regeneriert.

22. Transformierter Wirtsorganismus, insbesondere eine Pflanzenzelle oder Pflanze, **dadurch gekennzeichnet, daß** er/sie eine Nukleinsäuresequenz nach Anspruch 12 oder ein chimäres Gen nach einem der Ansprüche 13 bis 16 enthält.

23. Pflanzenzelle, **dadurch gekennzeichnet, daß** sie eine Nukleinsäuresequenz nach Anspruch 12 oder ein chimäres Gen nach einem der Ansprüche 13 bis 16 enthält.

24. Transformierte Pflanze, **dadurch gekennzeichnet, daß** sie transformierte Zellen nach Anspruch 23 enthält.

25. Pflanze nach Anspruch 24, **dadurch gekennzeichnet, daß** sie aus transformierten Zellen nach Anspruch 23 regeneriert wurde.

26. Transformierte Pflanze, **dadurch gekennzeichnet, daß** sie durch Kultur und/oder Kreuzung von regenerierten Pflanzen nach Anspruch 25 entstanden ist.

27. Transformierte Pflanzen nach einem der Ansprüche 24 bis 26, **dadurch gekennzeichnet, daß** sie aus der Reihe der einkeimblättrigen Pflanzen, insbesondere Getreide, Zuckerrohr, Reis und Mais, oder der zweikeimblättrigen Pflanzen, insbesondere Tabak, Sojabohne, Raps, Baumwolle, Zucker/Futterrübe und Klee stammen.

28. Transformierter Same, **dadurch gekennzeichnet, daß** er transformierte Zellen nach Anspruch 23 enthält und daß er von einer transformierten Pflanze nach einem der Ansprüche 24 bis 27 stammt.

29. Verfahren zur Bekämpfung von Unkräutern in einer Oberfläche eines Felds, das Samen nach Anspruch 28 oder transformierte Pflanzen nach einem der Ansprüche 24 bis 27 umfaßt, das darin besteht, daß man in die Oberfläche des Felds eine für diese Unkräuter toxische Dosis eines HPPD-Hemmer-Herbizids ausbringt, ohne jedoch die Samen oder Pflanzen, die mit dem erfindungsgemäßen chimären Gen transformiert wurden, wesentlich zu schädigen.

30. Verfahren zum Kultivieren von transformierten Pflanzen nach einem der Ansprüche 24 bis 27, das darin besteht, daß man die Samen dieser transformierten Pflanzen nach Anspruch 28 in die Oberfläche eines für die Kultur dieser Pflanzen geeigneten Felds sät, falls Unkräuter vorhanden sind in die Oberfläche des Felds eine für diese Unkräuter toxische Dosis eines HPPD-Hemmer-Herbizids ausbringt, ohne die Samen oder Pflanzen, die transformiert wurden, wesentlich zu schädigen und anschließend die kultivierten Pflanzen bei Erreichen der gewünschten Reife erntet und gegebenenfalls die Samen von den geernteten Pflanzen abtrennt.

31. Verfahren nach einem der Ansprüche 29 oder 30, **dadurch gekennzeichnet, daß** der HPPD-Hemmer aus der Reihe der Isoxazole, insbesondere Isoxaflutol, der Diketonitrile, insbesondere 2-Cyano-3-cyclopropyl-1-(2-SO₂CH₃-4-CF₃-phenyl)propan-1,3-dion und 2-Cyano-3-cyclopropyl-1- (2-SO₂CH₃-4-2, 3-Cl₂-phenyl) propan-1, 3-dion, der Triketone, insbesondere Sulcotrion, oder der Pyrazolinate stammt.
